# EUROPÄISCHE PATENTSCHRIFT

(11) **EP 2 417 385 B1**
(45) Veröffentlichungstag und Bekanntmachung des Hinweises auf die Patenterteilung: **01.07.2015**
(21) Anmeldenummer: 10713138.5
(22) Anmeldetag: 07.04.2010
(51) Int. Cl.: F16N 29/00, F16N 29/04, G01N 33/28, G07C 5/00, G08C 17/00, G08C 17/02

(54) **VERSCHLUSSVORRICHTUNG, GEHÄUSETEIL EINES SCHMIERMITTELBEHÄLTERS, DIAGNOSESYSTEM UND DIAGNOSEVERFAHREN ZUR ÜBERWACHUNG DES BETRIEBSZUSTANDS EINES SCHMIERMITTELS IN DEM GEHÄUSETEIL**
CLOSURE DEVICE, HOUSING PART OF A LUBRICANT CONTAINER, DIAGNOSTIC SYSTEM AND DIAGNOSTIC METHOD FOR MONITORING THE CONDITION OF THE LUBRICANT IN THE HOUSING PART
DISPOSITIF DE FERMETURE, PARTIE DE BOÎTIER D'UN RESERVOIR DE LUBRIFIANT, SYSTÈME DE DIAGNOSTIC ET PROCÉDÉ DE DIAGNOSTIC POUR SURVEILLER LA CONDITION DE SERVICE DU LUBRIFIANT DANS LA PARTIE DE BOÎTIER

(30) Priorität: 07.04.2009 US 167395 P; 07.04.2009 DE 102009016642
(43) Veröffentlichungstag der Anmeldung: 15.02.2012
(73) Patentinhaber: Airbus Operations GmbH, 21129 Hamburg (DE)
(72) Erfinder: DORR, Björn, 28816 Stuhr (DE); NUSCHELER, Franz, 81671 München (DE); PAUL, Sumit, 81925 München (DE)
(74) Vertreter: UEXKÜLL & STOLBERG
(86) Internationale Anmeldenummer: PCT/EP2010/002184
(87) Internationale Veröffentlichungsnummer: WO 2010/115618

(56) Entgegenhaltungen:
- EP-A1- 1 939 602
- WO-A1-01/90539
- US-A1- 2003 083 794
- US-A1- 2007 074 563
- US-A1- 2008 289 399
- US-A1- 2009 027 227
- US-B1- 6 392 562
- US-B1- 6 459 995

## Beschreibung

Die Erfindung betrifft eine Verschlussvorrichtung für einen Schmiermittelbehälter, ein Gehäuseteil eines Schmiermittelbehälters und ein Diagnosesystem zur Überwachung des Betriebszustands eines Schmiermittels in dem Gehäuseteil.

Die Erfindung betrifft insbesondere ein einen Schmiermittelbehälter teilweise oder ganz bildendes Gehäuseteil zur teilweisen oder vollständigen Aufnahme eines Leistungs-Übertragungsmechanismus, das mindestens eine Einlass- und/oder Auslassöffnung zum Auffüllen bzw. Ablassen eines Schmiermittels in das bzw. aus dem Gehäuseteil, wobei die Einlass- und Auslassöffnungen mit lösbaren Verschlussvorrichtungen abgedichtet sind.

Die EP 1 939 602 A1 beschreibt ein Wartungssystem zur Ermittlung eines Eisengehalts in einem Schmiermittel eines Getriebes.

Die US 2008/0289399 A1 offenbart einen Sensor mit Elektroden zur Ermittlung einer Qualität eines Fluids, die als in das Fluid hineinragende Elektroden ausgebildet sind.

Aus der US 6 392 562 B1 wird ein Sensor beschrieben, der zusammen mit einem Magneten, einem Partikel-Auffangraum und zwei Spulen zur Erfassung von Partikeln in einem Fluid eingesetzt wird.

Solche Gehäuseteile mit einem Schmiermittelreservoir finden beim Einbau in land-, luft-, wasser- oder unterwassergestützten Fahrzeugen Verwendung und dienen dort zur Aufnahme beweglicher mechanischer Komponenten, wie insbesondere Getriebe oder Drehaktuatoren, wobei diese beweglichen Komponenten in dem im Schmiermittelreservoir des Gehäuseteils untergebrachten Schmierfluid eingelagert sind und dadurch geschmiert und gegebenenfalls auch gekühlt werden.

Aus der DE 101 53 151 A1 ist ein System und ein Verfahren zur Wartung von Aktuatoren von Flugzeugen unter Verwendung von Sensoren zur Messung des Feuchtigkeitsgehalts in den Aktuatoren bekannt.

Aufgabe der Erfindung ist, eine Vorrichtung und ein Diagnosesystem bereitzustellen, mit der bzw. mit dem eine Erfassung oder Überwachung des Betriebszustands von Schmiermittel, das in mechanischen Geräten wie z.B. Aktuatoren verwendet wird, auf effiziente Weise möglich ist.

Diese Aufgabe wird mit den Merkmalen der unabhängigen Ansprüche gelöst. Weitere Ausführungsformen sind in den auf diese rückbezogenen Unteransprüchen angegeben.

Mit der erfindungsgemäßen Lösung ist es insbesondere möglich, die Auswirkungen von extremen Schwankungen der äußeren Umgebungsbedingungen, denen im Praxiseinsatz solche Gehäuseteile an Fahrzeugen ausgesetzt sind und bei denen es zu einer unerwünschten Einlagerung von Kondenswasser in das Schmiermittelreservoir oder anderen unerwünschten Reaktionen des Schmiermittels kommen kann und welche die Konsistenz der Schmiermittelzusammensetzung in negativer Weise beeinflussen können, zu überwachen.

Beispielsweise sei diese Problematik im Folgenden anhand von im Hochauftriebssystem eines Flugzeugs eingebauten Komponenten ausführlicher erläutert: Das Hochauftriebssystem eines Flugzeugs ist aus zahlreichen SystemKomponenten gebildet, die aufgrund ihrer Lage größtenteils den direkt den Umgebungsbedingungen ausgesetzt sind. Bei Variation der Flughöhe (d.h. Steigflug oder Sinkflug) ändern sich die dabei Umgebungsbedingungen wie Außentemperatur, Außenluftdruck und Luftfeuchtigkeit. Eine hermetische Abdichtung ist bei den meisten Systemkomponenten, so auch den Rotationsaktuatoren im Hochauftriebssystem, nicht möglich, weshalb häufig dynamische Dichtungen eingesetzt werden.

Diese Dichtungen können jedoch nicht verhindern, dass sich Temperatur, Luftdruck und Luftfeuchtigkeit im Inneren der Systemkomponenten ständig verändern. Diese Veränderungen treten jedoch abhängig von der Steig- und Sinkrate mehr oder weniger zeitverzögert zu den Veränderungen der Umgebungsbedingungen auf. Das bedeutet, dass während des Steigflugs der Luftdruck innerhalb einer Systemkomponente immer etwas höher ist als der Außenluftdruck und beim Sinkflug der Luftdruck innerhalb der Systemkomponente stets etwas niedriger ist als der Außenluftdruck.

Folglich wirkt beim Landeanflug ein erhöhter Druck auf die Komponente, sodass sich - vor allem in (tropischen und subtropischen) Gegenden mit hoher Luftfeuchtigkeit - Kondenswasser in den Systemkomponenten bildet. Beim Ausfahren des Hochauftriebssystems eines Flugzeugs werden Rotationsaktuatoren über ein Transmissionswellensystem angetrieben. Die Aktuatoren enthalten Schmiermittel vom Typ Semifluid (semifluide Fließfette). Werden beim Verstellen der Hochauftriebsklappen die Zahnräder im Inneren des Aktuators in Bewegung versetzt, und ist Kondenswasser in die Aktuatoren gelangt, so vermischt sich das Semifluid mit dem eingedrungenen Wasser. Mit der Anzahl der Flüge wiederholt sich dieser Prozess, sodass der Wasseranteil im Semifluid zunimmt. Ab einem gewissen Feuchtegrad besteht aufgrund der niedrigen Temperaturen in Reiseflughöhe die Gefahr der Eisbildung im Aktuator, was im schlimmsten Fall beim Ausfahren der Klappen zu einem Klemmfall führen kann. Tritt dieses Szenario ein, würde dem Piloten das Hochauftriebssystem beim Landeanflug nicht mehr zur Verfügung stehen.

Bisher wurde mit diesem Problem im Allgemeinen so umgegangen, dass das Semifluid unter Einhaltung bestimmter Wartungsintervalle regelmäßig ausgetauscht wurde. Bei der Wartung werden zunächst die Absperrschrauben an dem Gehäuseteil entfernt, woraufhin das Semifluid durch den unteren Auslass entweichen kann. Sobald kein Semifluid mehr ausströmt, wird die untere Absperrschraube inklusive der Dichtungsscheibe wieder montiert. Anschließend wird an der oberen Auslassöffnung ein Schlauch fixiert, dessen zweites Ende in einen Auffangbehälter führt. Nach Entfernung der Absperrschraube kann mithilfe einer Schmierpistole das neue Semifluid über das Einlassventil zugeführt werden. Nach erfolgreicher Befüllung mit der festgelegten Befüllungsmenge erfolgt das Schließen der Ein- und Auslassöffnungen durch die entsprechenden Absperrschrauben. Bei Nichteinhaltung der Wartungsintervalle oder bei einem erhöhten Feuchtegrad, wird das Semifluid zunehmend zähflüssiger, sodass ein Austausch nur noch durch Öffnen des Gehäusedeckels möglich ist. Der Wartungsaufwand ist dann jedoch derart zeitaufwendig, dass aus wirtschaftlichen Gründen häufig ein Komplettaustausch des Gehäuseteils erfolgt. Nachteilig bei dieser bekannten Vorgehensweise ist aus Wartungssicht, dass zur Vermeidung von Klemmfällen das Semifluid in regelmäßigen Abständen wie oben beschrieben ausgetauscht werden muss. Da der Wasseranteil im Semifluid abhängig von der Flugroute unterschiedlich schnell zunimmt, müsste der optimale Zeitpunkt für einen Semifluidwechsel aus wirtschaftlicher Sicht für jedes Flugzeug separat ermittelt werden. Diese Vorhersage ist jedoch äußerst schwierig, weshalb das Semifluid in festgelegten Wartungsintervallen ausgetauscht wird.

Erfolgt die Wartung verfrüht, so dass der Feuchteanteil bei nur wenigen Prozent liegt, so hätte die Fluggesellschaft die Kosten für die Wartung einsparen können. Bei zu groß gewählten Wartungsintervallen riskiert die Fluggesellschaft Schäden an Teilkomponenten oder gar einen Klemmfall im Aktuator, zudem kann das Semifluid bei erhöhtem Feuchtegrad derart zähflüssig werden, dass ein Austausch des Semifluids ohne ein zeitaufwendiges und folglich kostspieliges Öffnen des Rotationsaktuators - was meist zu einem Austausch der Komponente führt - nicht mehr möglich ist.

Mit der erfindungsgemäßen Lösung kann der zuvor beschriebene Wartungsaufwand vermieden oder deutlich eingeschränkt werden. Neben der Verwendung der erfindungsgemäßen Lösung wie beschrieben für einen Flap-Rotationsaktuator für die Verstellung einer Auftriebshilfe(Landeklappe) an der Tragfläche eines Flugzeugs zur Überwachung der Kondenswasser-Einlagerung in diesem Flap-Rotationsaktuator kann die erfindungsgemäße Lösung in ähnlicher Weise auch bei an sich beliebigen Gehäuseteilen mit beweglichen mechanischen Komponenten in einem Schmiermittelreservoir in beliebigen land-, luft-, wasser- oder unterwassergestützen Fahrzeugen (z.B. Kraftfahrzeuge, Flugzeuge, Helikopter, Schiffe, U-Boote, etc.) vorgesehen sein. Die Verwendung der erfindungsgemäßen Lösung kann insbesondere dort vorgesehen sein, wo es beim Einsatz eines solchen Fahrzeugs aufgrund äußerer Umwelteinflüsse zu unerwünschten Veränderungen der Zusammensetzung und der Eigenschaften des Schmiermittels kommen kann, so dass die Schmiermittelzusammensetzung regelmäßig zu überwachen und das Schmiermittel gegebenenfalls rechtzeitig zu ersetzen ist. Die Erfindung kann also generell zum Einbau in ein Fahrzeug vorgesehenen Gehäuseteilen mit darin in einem Schmiermittelreservoir gelagerten beweglichen Komponenten, wie z.B. Getrieben oder Drehaktuatoren, vorgesehen sein. Es wird ein Aufbau vorgeschlagen, bei dem eine regelmäßige Diagnose der Konsistenz des äußeren Umwelteinflüssen ausgesetzten Schmiermittels auf möglichst einfache Weise durchgeführt werden kann, und dass dann, wenn eine solche Diagnose zum Ergebnis führt, dass ein Austausch des Schmiermittels zu erfolgen hat, dieser Austauschvorgang auf möglichst einfache Weise durchgeführt werden kann. Auch wird ein Diagnosesystem bereitgestellt, das eine möglichst einfache Überwachung des Betriebszustands des Schmiermittels im Schmiermittelreservoir in einem Gehäuseteil mit beweglichen mechanischen Komponenten ermöglicht.

Erfindungsgemäß ist eine Verschlussvorrichtung zur Abdichtung einer Öffnung eines Gehäuseteils gegen ein Schmiermittel, die einen Innenraum aufweist, vorgesehen, wobei der Innenraum der Verschlussvorrichtung aufweist:
- zumindest eine Sensorvorrichtung mit einem Sensor zur Erzeugung von dem Betriebszustand des Schmiermittels entsprechenden Sensorsignalen,
- zumindest einen Transceiver zum Empfang der Sensorsignale von der Sensorvorrichtung und zur Signalübertragung der Sensorsignale an eine externe Empfangseinheit,
- eine Energieversorgungsvorrichtung zur Versorgung der Sensorvorrichtung und des Transceivers mit elektrischer Energie,
wobei die Oberfläche eines Endabschnitts als Sensoroberfläche zur Erfassung der Sensorwerte ausgebildet ist und wobei die Sensorvorrichtung einen Planar-Kondensator zur Erfassung des Wassergehalts des Schmiermittels aufweist, der aus zwei einander zugewandten Kondensator-Elektroden gebildet ist.

Durch das Vorsehen einer Sensorvorrichtung in einer Verschlussvorrichtung kann die Sensorvorrichtung mit der Verschlussvorrichtung ohne weiteres ausgetauscht werden und ist für das Einsetzen der Verschlussvorrichtung in den für diese vorgesehenen Ort, z.B. eine Öffnung eines Gehäuseteils, keine gesonderte und aufwändige Integrations- und Kontrolltätigkeit erforderlich. Auch ist der logistische Aufwand für Ersatzteile für die Sensorvorrichtung einfach, da die Verschlussvorrichtungen ohne weiteres austauschbar sind.

Die erfindungsgemäße Verschlussvorrichtung kann als Schraube und zum Einsetzen in eine Gewindeaufnahme einer Einlassöffnung oder Auslassöffnung oder als Bolzen mit einem Verankerungselement zum Verschließen z.B. einer jeweiligen Einlassöffnung oder einer Auslassöffnung mit dem Bolzen ausgebildet sein.

Der Transceiver kann insbesondere zur Herstellung einer Funkübertragungsverbindung zur Übertragung der Sensorsignale an eine externe Empfangseinheit ausgebildet und an eine Antenne gekoppelt sein, die an einer Oberfläche eines zweiten, entgegen gesetzt zu dem ersten Endabschnitt gelegenen Endabschnitts angeordnet ist.

Weiterhin kann vorgesehen sein, dass die Sensorvorrichtung zusätzlich einen Temperatursensor zur Erfassung der Temperatur des Schmiermittels und/oder einen Drucksensor zur Erfassung des Druckes des Schmiermittels aufweist. Insbesondere kann eine Kombination der Messung des Wassergehalts und der Temperatur des Schmiermittels vorgenommen werden. Dadurch kann die Messung des Wassergehaltes genauer vorgenommen werden. Die in der Sensorvorrichtung integrierte Messfunktion ermittelt in diesem Ausführungsbeispiel den Wassergehalt in Abhängigkeit der ebenfalls gemessenen Temperatur des Schmiermittels.

Nach einem weiteren Ausführungsbeispiel kann die Sensorvorrichtung eine Messfunktion aufweisen, in der zumindest eine zeitliche Vorgabe für die Durchführung einer Messung und eine Funktion zur Durchführung der Messung eingerichtet sind, die bei Erfüllung der zeitlichen Vorgabe den Sensor aktiviert und Sensorsignale zur Ermittlung von Sensorwerten erfasst. Die zeitliche Vorgabe kann durch einen Zeitgeber der Sensorvorrichtung realisiert sein, der mit der Messfunktion derart gekoppelt ist, dass die Messfunktion auf ein Aktivierungssignal des Zeitgebers eine Messung des Betriebszustands des Schmiermittels durchführt. Der Messwert für den Betriebszustand des Schmiermittels wird dabei in einer Speichervorrichtung abgespeichert, von der der Messwert auf Abfrage z.B. durch eine externe Wartungsvorrichtung ausgelesen und an die Wartungsvorrichtung übermittelt werden kann. Dieses Ausführungsbeispiel hat den Vorteil, dass die Verschlussvorrichtung mit der Sensorvorrichtung auf sehr einfache Weise realisiert werden kann. Auch können die Sensorfunktionen zur Messung unabhängig von externen Vorrichtungen wie z.B. der Wartungsvorrichtung operativ sein. Dadurch braucht die Messfunktion zur Durchführung der Messung insbesondere keine Schnittstelle zu einer externen Vorrichtung aufweisen.

Alternativ oder zusätzlich kann nach einem Ausführungsbeispiel der Transceiver eine Ansteuerungsvorrichtung aufweisen, die mit der Sensorvorrichtung funktional in Verbindung steht und die auf Empfang eines Messkommandos zur Durchführung einer Messung die Messfunktion der Sensorvorrichtung mittels eines Aktivierungssignals aktiviert, so dass die Sensorvorrichtung zumindest ein Sensorsignal zur Ermittlung von Sensorwerten erfasst und dieses an den Transceiver übermittelt.

Die Sensorvorrichtung weist den Planar-Kondensator auf oder ist aus diesem gebildet, der aus zwei einander zugewandten Kondensatorteilen gebildet ist. Es ist insbesondere vorgesehen, dass die zur Messung des Betriebszustands des Schmiermittels die zur Kontaktierung mit dem Schmiermittel vorgesehene Sensoroberfläche derart an der Verschlussvorrichtung angeordnet ist, dass diese im schließenden Zustand der Verschlussvorrichtung dem Inneren des Gehäuseteiles zugewandt ist.

Nach einem weiteren Aspekt der Erfindung ist ein Gehäuseteil eines Schmiermittelbehälters vorgesehen, das eine Verschlussvorrichtung nach der Erfindung mit einer Sensorvorrichtung aufweist, die zur Erzeugung von dem Betriebszustand des in dem Gehäuseteil enthaltenen Schmiermittels entsprechenden Sensorsignalen ausgebildet ist. Insbesondere kann vorgesehen sein, dass das Gehäuseteil zumindest eine Öffnung und eine darin einsetzbare Verschlussvorrichtung mit einer Sensorvorrichtung nach der Erfindung aufweist. Die Verschlussvorrichtung kann insbesondere nach einem der hierin beschriebenen Ausführungsbeispiele gebildet sein. Weiterhin kann das Gehäuseteil und die Verschlussvorrichtung derart gestaltet sein, dass die Verschlussvorrichtung in ihrem das Gehäuseteil schließenden Zustand mit einer Oberfläche eines ersten Endabschnitts dem Inneren des Gehäuseteils zugewandt ist, um Kontakt mit dem Schmiermittel zu haben, wobei die Oberfläche als Sensoroberfläche zur Erfassung der Sensorwerte ausgebildet ist.

Nach einem weiteren Ausführungsbeispiel der Erfindung ist vorgesehen, dass das Gehäuseteil wenigstens eine Auslassöffnung oder Einlassöffnung zum Auslassen des Schmiermittels aus dem Gehäuse oder Schmiermittelbehälter im zusammengesetzten Zustand aufweist, dessen Teil das Gehäuseteil ist, und dass die wenigstens eine Auslassöffnung oder Einlassöffnung mit jeweils der lösbaren Verschlussvorrichtung abgedichtet ist. Die Verschlussvorrichtung als Schraube ausgebildet sein und die jeweilige Öffnung des Gehäuseteils kann eine Gewindeaufnahme zur Aufnahme der Schraube aufweisen. Auch kann die Verschlussvorrichtung als Bolzen mit einem Verankerungselement zum Verschließen der jeweiligen Einlass- und Auslassöffnung mit dem Bolzen ausgebildet sein. Durch das Vorsehen oder Einsetzen einer erfindungsgemäßen Verschlussvorrichtung in einer Auslassöffnung oder Einlassöffnung des Gehäuseteils eines Schmiermittelbehälters kann eine bereits ohnehin zu einem gegebenenfalls anderen Zweck vorgesehene Öffnung zusätzlich zur Integration einer Sensorvorrichtung nach der Erfindung vorgesehen sein. Auf diese Weise ist kein gesonderter mechanischer Integrationsaufwand für die Integration der erfindungsgemäßen vorgesehenen Sensorvorrichtung in das Gehäuseteil bzw. den Schmiermittelbehälter notwendig.

Das einen Schmiermittelbehälter bildende Gehäuseteil nach der Erfindung kann insbesondere ein Gehäuseteil eines Leistungs-Übertragungsmechanismus sein. Der Übertragungsmechanismus kann insbesondere ein Getriebe sein. Weiterhin kann das Getriebe das Getriebe eines Drehaktuators sein.

Nach einem weiteren Aspekt der Erfindung ist ein Diagnosesystem vorgesehen, aufweisend: ein Gehäuseteil nach einem der hierin beschriebenen Ausführungsbeispiele und eine Wartungsvorrichtung mit einem Transceiver zum Empfang von Sensorsignalen von dem der Sensorvorrichtung zugeordneten Transceiver, wobei die Wartungsvorrichtung eine Verarbeitungsfunktion zur Ermittlung eines Wertes für den Betriebszustand eines in dem Gehäuseteil befindlichen Schmiermittels aus dem Sensorsignal aufweist. Nach einem Ausführungsbeispiel ist die Wartungsvorrichtung in dem Fahrzeug, in dem auch der Schmiermittelbehälter mit dem Gehäuseteil integriert ist, lösbar oder fest installiert. Alternativ oder zusätzlich kann vorgesehen sein, dass die Wartungsvorrichtung eine mobile Wartungsvorrichtung ist, die insbesondere als Handgerät ausgebildet sein kann.

Bei dem Diagnosesystem kann vorgesehen sein, dass die Sensorvorrichtung eine Messfunktion aufweist, in der zumindest eine zeitliche Vorgabe für die Durchführung einer Messung und eine Funktion zur Durchführung der Messung eingerichtet sind, die bei Erfüllung der zeitlichen Vorgabe den Sensor aktiviert und Sensorsignale zur Ermittlung von Sensorwerten erfasst, dass die Wartungsvorrichtung eine mit dem Transceiver funktional verbundene Eingabevorrichtung zur Abfrage eines Sensorwertes aufweist, die auf eine Betätigung der Eingabevorrichtung den Transceiver der Verschlussvorrichtung ansteuert und diesen zum Senden eines dem erfassten Sensorsignal entsprechenden Signals aktiviert und dieses empfängt. In diesem Ausführungsbeispiel kann insbesondere vorgesehen sein, dass Messsignale aufgrund des Zeitgebers durch die Messfunktion erzeugt und in der Speichervorrichtung abgespeichert werden. Auf das Aktivierungssignal durch die Wartungsvorrichtung wird das Messsignal oder ein daraus ermittelter Sensorwert über die Transceiver an die Wartungsvorrichtung übermittelt, an der dieser zur Anzeige gebracht werden kann.

Weiterhin kann vorgesehen sein, dass die Sensorvorrichtung derart eingerichtet ist, auf Empfang eines Steuerungskommandos von dem der Sensorvorrichtung zugeordneten Transceiver ein einem Betriebszustand des Schmiermittels entsprechendes Sensorsignal erfasst und an die Wartungsvorrichtung sendet.

Nach einem Ausführungsbeispiel des erfindungsgemäßen Diagnosesystems ist vorgesehen, dass die Sensorvorrichtung aufweist: eine mit der Verarbeitungsfunktion zur Ermittlung eines Wert für den Betriebszustand eines in dem Gehäuseteil befindlichen Schmiermittels aus dem erfassten Sensorsignal und eine funktional mit der Verarbeitungsfunktion verbundene Diagnosefunktion zur Ermittlung von Wartungsinformationen aus dem jeweils ermittelten Wert für den Betriebszustand eines in dem Gehäuseteil befindlichen Schmiermittels. Auf diese Weise braucht die Wartungsfunktion keine sensorspezifische Auswertung- oder Diagnosefunktion aufweisen. Falls der Sensor ausgewechselt wird, muss die Wartungsvorrichtung nicht angepasst zu werden, da diese in diesem Fall keine spezifische Auswertung- und Diagnosefunktion aufweist. Auch kann die Auswertungs- oder Diagnosefunktion in der Sensorvorrichtung auf jeden einzelnen Sensor angepasst werden, z.B. um die Sensorauswertung oder den spezifischen Sensor zu kalibrieren oder anzugleichen. Alternativ kann die Diagnosefunktion in der Wartungsvorrichtung integriert sein.

Die Wartungsvorrichtung kann eine Anzeigevorrichtung zur Anzeige der Sensorgrößen und/oder von ermittelten Wartungsinformationen aufweisen. Auch kann die Wartungsvorrichtung ein Wartungs-Handgerät sein, in dem die Verarbeitungsfunktion und die Anzeigevorrichtung strukturell sind.

Nach einem weiteren Aspekt der Erfindung ist ein Diagnosesystem mit einer Wartungsvorrichtung nach einer der erfindungsgemäßen Ausführungsformen vorgesehen, wobei die Wartungsvorrichtung eine mit einer ausgeführten Sensorvorrichtung zur Zuführung von Sensorsignalen funktional in Verbindung stehende Vergleichsfunktion aufweist, die derart ausgeführt ist, dass die Vergleichsfunktion das jeweils erfasste Messsignal mit zwei Grenzwerten vergleicht und aufgrund dieses Vergleichs identifiziert, ob das jeweils erfasste Signalwert in einem ersten Bereich unterhalb eines ersten Grenzwerts oder in einem zweiten Bereich zwischen dem ersten und einem zweiten Grenzwert, der größer als der erste Grenzwert ist, oder in einem dritten Bereich oberhalb des zweiten Grenzwertes gelegen ist. Die Wartungsvorrichtung weist dabei eine Anzeigefunktion auf, die aufgrund der Identifizierung eines Bereichs für den jeweils erfassten Signalwert eine Markierung eines von drei Feldern, die jeweils einem der Bereich zugeordnet sind, in einem Anzeigeformat der Anzeigevorrichtung vornimmt. Die Vergleichsfunktion kann auch in der Sensorvorrichtung integriert sein, so dass die Sensorvorrichtung eine Information über die Zuordnung des in dieser ermittelten Signalwertes zu dem ersten, zweiten oder dritte Bereich ermittelt, die an die Anzeigevorrichtung übermittelt wird und von dieser zur Anzeige gebracht wird. Beispielsweise kann der dritte Bereich einen kritischen Bereich kennzeichnen, dessen Zutreffen die Vornahme des Austausches der Komponente anzeigen soll, in dem die Sensorvorrichtung integriert ist. Der erste Bereich kann dabei einen zulässigen Bereich kennzeichnen, dessen Zutreffen anzeigen soll, dass die Komponente sich in einem zulässigen Betriebszustand befindet und keine Wartungsmaßnahme notwendig ist. Der zweite Bereich kann dabei einen zulässigen Bereich kennzeichnen, dessen Zutreffen anzeigen soll, dass die Komponente sich in einem zwar zulässigen, jedoch in einem Betriebszustand befindet, bei dem sich eine Wartungsmaßnahme ankündigt oder eine spezifische Wartungsmaßnahme notwendig ist.

Die Zuordnung an den ersten und/oder zweiten Bereich und die Anzeige des Vorliegens des ersten und/oder zweiten Bereichs kann auch entfallen. Demgemäß kann die erfindungsgemäße Wartungsvorrichtung eine mit der Sensorvorrichtung zur Zuführung von Sensorsignalen funktional in Verbindung stehende Vergleichsfunktion aufweisen, die derart ausgeführt ist, dass die Vergleichsfunktion das jeweils erfasste Messsignal mit zumindest einem Grenzwert vergleicht und aufgrund dieses Vergleichs identifiziert, ob das jeweils erfasste Signalwert in einem ersten Bereich unterhalb dieses Grenzwerts oder in einem zweiten Bereich oberhalb dieses Grenzwertes gelegen ist, und dass die Wartungsvorrichtung eine Anzeigefunktion aufweist, die aufgrund der Identifizierung eines Bereichs für den jeweils erfassten Signalwert eine Markierung zumindest eines Felds oder eines von zwei Feldern, die jeweils einem der Bereiche zugeordnet sind, in einem Anzeigeformat der Anzeigevorrichtung vornimmt. Bei Vorsehen nur eines Bereichs bei der Anzeigevorrichtung und der zugehörigen Funktionalität ist dieser Bereich insbesondere einen vorgenannten kritischen Bereich kennzeichnen. Bei vorsehen von zwei Bereichen der Anzeigevorrichtung und der zugehörigen Funktionalität ist kann einer dieser Bereich insbesondere einen vorgenannten kritischen Bereich und der weitere Bereich einen zulässigen Bereich kennzeichnen.

Weiterhin kann vorgesehen sein, dass die Wartungsvorrichtung ein Funktionsmodul aufweist, mit dem ein Wert für einen Betriebszustand eines in dem Gehäuseteil befindlichen Schmiermittels selektiert werden kann, mit dem eine für eine Wartungsaufgabe notwendige Wartungsinformation an eine Anzeigeeinrichtung übermittelt und mit der Anzeigeeinrichtung angezeigt werden kann.

Nach einem weiteren Ausführungsbeispiel des erfindungsgemäßen Diagnosesystems ist vorgesehen, dass in dem Gehäuseteil mindestens zwei erfindungsgemäße Verschlussvorrichtungen integriert sind, dass die Wartungsvorrichtung eine Vergleichsfunktion aufweist, die mit dem Transceiver der Wartungsvorrichtung funktional verbunden ist und aus den Sensorsignalen von zwei verschiedenen Sensorvorrichtungen ein Vergleichsfunktionswert gebildet wird, der als Wert für den Betriebszustand eines in dem Gehäuseteil befindlichen Schmiermittels verwendet wird. Auch kann vorgesehen sein, dass die Vergleichsfunktion derart ausgeführt ist, dass diese den jeweils größten Signalwert der verschiedenen Sensorvorrichtung eines Schmiermittelbehälters als ermittelter und anzuzeigender Signalwert verwendet. Dieses Ausführungsbeispiel hat den Vorteil, dass die Sicherheit, mit der der Schmiermittelzustand ermittelt und angezeigt wird, erhöht wird. Dabei können sowohl lokale Unterschiede des Betriebszustands des Schmiermittels wie auch Fehler an der Sensorvorrichtung oder einem Bestandteil der Verschlussvorrichtung kompensiert werden.

Die Transceiver der Wartungsvorrichtung und der zur Verschlussvorrichtung gehörende Transceiver können kabellos oder über eine Kabelverbindung miteinander funktional in Verbindung stehen.

Mit Hilfe des erfindungsgemäßen Diagnosesystems ist die Durchführung eines Diagnoseverfahrens zur Unterstützung der Wartung eines land-, luft-, wasser- oder unterwassergebundenen Fahrzeugs ausführbar, aufweisend die Schritte:
- Erzeugung eines Sensorsignals zum Erfassen eines Betriebszustands eines in dem Gehäuseteil enthaltenen Schmiermittels mittels der in der Verschlussvorrichtung des Gehäuseteils integrierten Sensorvorrichtung und Übermittlung des dem Betriebszustand entsprechenden Sensorsignals an die Wartungsvorrichtung,
- aufgrund des an die Wartungsvorrichtung übermittelten Sensorsignals in der Wartungsvorrichtung Ermittlung eines Wertes für den Betriebszustand eines in dem Gehäuseteil befindlichen Schmiermittels aus dem Sensorsignal,
- aufgrund einer Betätigung der Wartungsvorrichtung Übermittlung eines Kommandosignals an den in das Gehäuseteil insbesondere eines zu überwachenden Leistungs-Übertragungsmechanismus integrierten Transceivers und Empfang eines dem Betriebszustand des Schmiermittels entsprechenden Sensorsignals durch die Wartungsvorrichtung und Anzeige des Betriebszustands des Schmiermittels durch die Wartungsvorrichtung.

Dabei kann vorgesehen sein, dass die Sensorvorrichtung selbst und insbesondere auf Aktivierung des Zeitgebers ein dem Betriebszustand des Schmiermittels entsprechendes Sensorsignal erfasst, daraus ein dem Betriebszustand des Schmiermittels entsprechenden Sensorwert ermittelt, den Sensorwert in einem Speicher abspeichert und den Sensorwert auf ein von der Wartungsvorrichtung empfangenes Anforderungskommando an die Wartungsvorrichtung übermittelt.

Alternativ oder zusätzlich kann vorgesehen sein, dass die Wartungsvorrichtung an die Sensorvorrichtung ein Anforderungskommando zur Übermittlung eines dem Betriebszustand des Schmiermittels entsprechenden Sensorwerts sendet, Ermittlung des Sensorwertes durch die Sensorvorrichtung und Übermittlung desselben an die Wartungsvorrichtung.

Im Rahmen eines solchen Diagnoseverfahrens kann vorgesehen sein, dass in der Wartungsvorrichtung eine Wartungsfunktion integriert ist, die aus dem Wert für den Betriebszustand eines in dem Gehäuseteil befindlichen Schmiermittels Wartungsinformationen ermittelt. Auch kann vorgesehen sein, dass die Wartungsvorrichtung die ermittelten Wartungsinformationen mit einer Anzeigevorrichtung anzeigt.

Insbesondere ist bei dem Diagnoseverfahren vorgesehen, dass bei der Ermittlung des Wertes für den Betriebszustand eines in dem Gehäuseteil befindlichen Schmiermittels aus einem Sensorsignal ein Wert für den Wassergehalt im Schmiermittelbad aus einem von der Sensorvorrichtung erfassten Signalwert ermittelt wird.

Es kann weiterhin vorgesehen sein, dass an einem Überprüfungsort mindestens zwei Sensoreinrichtungen eingesetzt werden, deren Sensorsignale an die Verarbeitungseinheit übermittelt und dort miteinander verglichen werden, wobei als Ergebnis des Vergleichs ein Vergleichsfunktionswert gebildet wird, der weiteren Verarbeitung zugrunde gelegt wird. Weiterhin kann vorgesehen sein, dass die zentrale Verarbeitungseinheit die von einer Sensorbaugruppe empfangenen Signale mit vorgegebenen Grenzwerten für diese Signale vergleicht und auf einer Anzeige visualisiert, ob das empfangene Signal innerhalb vorgegebener Grenzwerte für das Signal liegt. Das Diagnoseverfahren kann derart ausgeführt sein, dass wenn ein empfangenes Signal außerhalb des Bereichs der vorgegebenen Grenzwerte liegt, die Verarbeitungseinheit ein Warnsignal, insbesondere ein akustisches oder optisches Warnsignal, ausgibt. Bei der Ausgestaltung des Diagnoseverfahrens oder des Diagnosesystems derart, dass die zentrale Verarbeitungseinheit Signale von mehreren Sensorbaugruppen empfängt, kann vorgesehen sein, dass auf der Anzeigevorrichtung die relative Position der Sensorbaugruppen zueinander und/oder relativ zum Fahrzeugkörper eines land-, luft-, wasser- oder unterwassergebundenen Fahrzeugs, in welchem die Sensorbaugruppen eingebaut sind, angezeigt wird.

Bei dem Diagnoseverfahren kann weiterhin insbesondere vorgesehen sein, dass in der Wartungsvorrichtung aus den Sensorsignalen von zwei verschiedenen jeweils in dem Gehäuseteil integrierten Verschlussvorrichtungen ein Vergleichsfunktionswert gebildet wird, der als Wert für den Betriebszustand eines in dem Gehäuseteil befindlichen Schmiermittels verwendet wird, wobei als Vergleichfunktionswert insbesondere jeweils der größte der in einem Messzeitraum ermittelten Messwerte verwendet wird.

Bei dem Diagnoseverfahren kann die Übermittlung eines Kommandosignals von der Wartungsvorrichtung an den in dem Gehäuseteil integrierten Transceiver und die Übermittlung des Sensorsignals von dem Transceiver an die Wartungsvorrichtung kabellos erfolgt.

Es kann auch vorgesehen sein, dass in der Sensorvorrichtung oder, bei Verwendung mehrerer Verschlussvorrichtungen in einem Schmiermittelbehälter, in den mehreren Sensorvorrichtungen jeweils ein Satellitenpositions-Sensor integriert ist. Mit diesem wird die Position der Verschlussvorrichtung ermittelt. Dabei kann weiterhin vorgesehen sein, dass diese in der Sensorvorrichtung und insbesondere in einer Speichervorrichtung derselben abgespeichert wird und auf Abfrage optional zusammen jeweils mit einem Sensorwert an die Wartungsvorrichtung übermittelt wird.

Bei dem Diagnoseverfahren kann vorgesehen sein, dass an wartungsintensiven Teilsystemen bzw. Komponenten des Fahrzeugs an einem Überprüfungsort in mindestens einer Sensorvorrichtung Sensorsignale erzeugt und/oder gespeichert werden, die Sensorsignale an die Wartungsvorrichtung übermittelt werden, die aus den Sensorsignalen verschiedener Teilsysteme einen Betriebszustand ermittelt.

Nach einem weiteren Aspekt der Erfindung ist ein Land-, luft-, wasser- oder unterwassergestütztes Fahrzeug vorgesehen, in welches ein erfindungsgemäßes Gehäuseteil und/oder ein erfindungsgemäßes Diagnosesystem eingebaut ist.

Die Vorteile und Merkmale der vorliegenden Erfindung ergeben sich auch aus den nachfolgenden Ausführungsbeispielen in Verbindung mit den Zeichnungen, die zeigen:
- Figur 1 eine schematische Schnittdarstellung eines in einem als Schmiermittelbehälter ausgebildeten Gehäuseteil untergebrachten Leistungs-Übertragungsmechanismus in Form eines Getriebes, wobei das Gehäuseteil erfindungsgemäß eine Auslassöffnung aufweist, die mit einem Verschlusselement geschlossen ist;
- Figur 2 eine schematische Draufsicht auf ein Ausführungsbeispiel eines Rotationsaktuators mit einer Grundplatte und einem als Schmiermittelbehälter ausgebildeten Gehäuseteil, in dem ein aus einem Getriebe gebildeter Leistungs-Übertragungsmechanismus aufgenommen ist;
- Figur 3 eine schematische Darstellung eines Ausführungsbeispiels eines erfindungsgemäß vorgesehenen Verschlusselements mit einem als Kondensator ausgebildeten Endabschnitt, in den eine Sensorvorrichtung und ein Transceiver sowie eine Energieversorgung für den Kondensator funktional integriert sind und wobei der Kondensator als Planar-Kondensator ausgebildet ist;
- Figur 4 eine schematische Darstellung des als Kondensator ausgebildeten und einen Planar-Kondensator aufweisenden Endabschnitts des in der Figur 3 dargestellten Kondensators,
- Figur 5 eine schematische Darstellung eines keine Ausführungsform der Erfindung darstellenden, sondern das Verständnis der Erfindung erleichternden Beispiels eines Verschlusselements mit einem als Kondensator ausgebildeten Endabschnitt, in den eine Sensorvorrichtung und ein Transceiver sowie eine Energieversorgung für den Kondensator funktional integriert sind, wobei der Kondensator mit ringförmigen Kondensatorflächen ausgebildet ist;
- Figur 6 eine schematische Darstellung eines weiteren keine Ausführungsform der Erfindung darstellenden, sondern das Verständnis der Erfindung erleichternden Beispiels eines Verschlusselements mit einem als Kondensator ausgebildeten Endabschnitt, in den eine Sensorvorrichtung und ein Transceiver sowie eine Energieversorgung für den Kondensator funktional integriert sind, wobei der Kondensator als Platten-Kondensator ausgebildet ist;
- Figur 7a ein Ersatzschaltbild für eine elektrische Ansteuerung eines in den Figuren 3, 5 und 6 dargestellten Plattenkondensators;
- Figur 7b ein weiteres Ersatzschaltbild für eine elektrische Ansteuerung eines in den Figuren 3, 5 und 6 dargestellten Plattenkondensators;
- Figur 8a ein Ausführungsbeispiel einer für das erfindungsgemäße Diagnosesystem verwendbaren Wartungsvorrichtung, die als Handgerät ausgebildet ist, mit einem Ausführungsbeispiel einer Diagnoseanzeige,
- Figur 8b ein gegenüber den mit den Figuren 6a und 6b dargestellten Anzeigeoptionen weiteres Ausführungsbeispiel einer Diagnoseanzeige einer für das erfindungsgemäße Diagnosesystem verwendbaren und als Handgerät ausgebildeten Wartungsvorrichtung, wobei die beispielsweise dargestellten Anzeigeinhalte für den Einsatz bei der Wartung eines Flugzeugs vorgesehen ist.

Die erfindungsgemäße Verschlussvorrichtung, das erfindungsgemäße Gehäuseteil und das erfindungsgemäße Diagnosesystem werden im folgenden insbesondere auch am Beispiel eines Flugzeugs, und dort insbesondere anhand der Problematik der Ermittlung der Konzentration von Wassereinlagerungen in einem Schmiermittelreservoir erläutert.

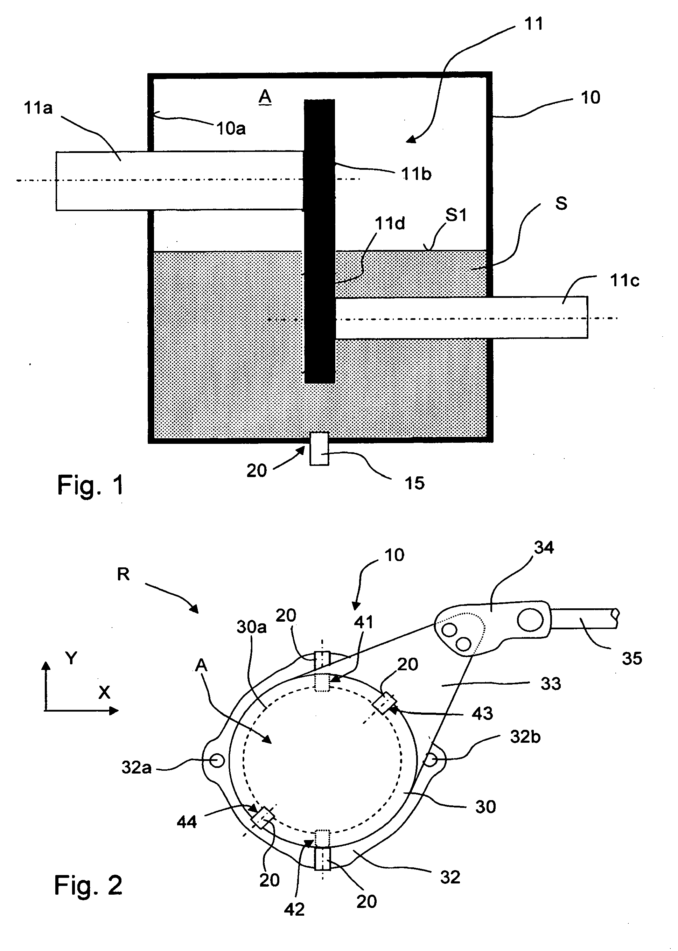

Nach einem Aspekt der Erfindung ist eine Verschlussvorrichtung 20 zur Abdichtung einer Öffnung eines Gehäuseteils 10 gegen ein Schmiermittel vorgesehen, die als Schraube oder Bolzen gestaltet ist und in die integriert sind (Figur 1): eine Sensorvorrichtung zur Erzeugung von dem Betriebszustand des Schmiermittels entsprechenden Sensorsignalen sowie einen Transceiver zur Signalübertragung der Sensorsignale an eine externe Empfangseinheit. Dabei ist eine Oberfläche der Verschlussvorrichtung 20 als Sensoroberfläche zur Erfassung der Sensorwerte ausgebildet ist.

Nach einem weiteren Aspekt der Erfindung ist ein Gehäuseteil eines Schmiermittelbehälters mit einer solchen Verschlussvorrichtung zur Abdichtung oder zum Verschließen einer Einlass- oder Auslassöffnung des Schmiermittelbehälters vorgesehen. Das Gehäuseteil kann generell Teil eines Schmiermittelbehälters sein oder diesen vollständig ausbilden. Der Schmiermittelbehälter mit dem erfindungsgemäßen Gehäuseteil 10 kann generell zur Erfüllung einer Reservoir- oder Aufbewahrungsfunktion vorgesehen sein. Alternativ oder zusätzlich kann der Schmiermittelbehälter mit dem erfindungsgemäßen Gehäuseteil 10 zur Aufnahme eines Leistungs-Übertragungsmechanismus 11 und dabei auch als Schmiermittelbehälter zur Aufnahme des zur Schmierung des Leistungs-Übertragungsmechanismus 11 erforderlichen Schmiermittels ausgebildet sein.

In der Figur 1 ist ein erfindungsgemäßes Gehäuseteil 10 mit einem darin angeordneten Leistungs-Übertragungsmechanismus 11 schematisch dargestellt. Diese Schnittdarstellung zeigt den in dem Gehäuseteil untergebrachten Leistungs-Übertragungsmechanismus 11 in Form eines Getriebes mit einer Eingangswelle 11a, einem daran angeordneten und als Zahnrad ausgebildeten Übertragungsrad 11 b, einer Ausgangswelle 11c und einem daran angeordneten und als Zahnrad ausgebildeten zweiten Übertragungsrad oder Abtriebsrad 11 b. In dem Gehäuseteil 10, das bei der Darstellung der Figur 1 einteilig ausgebildet ist, befindet sich zur Schmierung des Übertragungsmechanismus 11 ein Schmiermittel S oder ein Schmierfluid, das im betrieblichen Einsatz insbesondere in flüssigem Zustand vorliegt.

Erfindungsgemäß weist das Gehäuseteil 10 wenigstens eine Einlassöffnung und/oder wenigstens eine Auslassöffnung zum Auffüllen des Schmiermittels S in den Schmiermittelbehälter bzw. zum Ablassen des Schmiermittels aus demselben auf, die jeweils mit einer lösbaren Verschlussvorrichtung 20 abgedichtet ist. Das in der Figur 1 dargestellte Gehäuseteil 10 weist eine Auslassöffnung 15 zum Auslassen des Schmiermittels S auf, in die die Verschlussvorrichtung 20 eingesetzt ist, so dass bei Herausnehmen der Verschlussvorrichtung 20 aus der Auslassöffnung 15 diese geöffnet ist und durch diese das Schmiermittel aus dem Gehäuseteil 10 entweichen kann.

Dabei kann vorgesehen sein, dass für den bestimmungsgemäßen Betrieb des Gehäuses bzw. des Gehäuseteils das Schmiermittel das Gehäuse bzw. das Gehäuseteil 10 nicht vollständig ausfüllt, so dass sich ein Oberflächenspiegel S1 des Schmiermittels S in dem Gehäuse bzw. dem Gehäuseteil 10 ausbildet. In diesem Fall ist eine Öffnung des erfindungsgemäßen Gehäuseteils 20, in die eine erfindungsgemäße Verschlussvorrichtung mit einer Sensorvorrichtung eingesetzt ist, in einem Bereich unterhalb des Oberflächenspiegel S1 des Schmiermittels S des Gehäuses mit dem Gehäuseteil 10 nach der Erfindung gelegen, wenn das Gehäuse bzw. das Gehäuseteil 10 an seinem Bestimmungsort eingebaut oder installiert ist, so dass die Öffnung bzw. die Sensorvorrichtung im Betrieb des Gehäuse bzw. des Gehäuseteils 10 im Normalzustand in oder an einem mit Schmiermittel angefüllten Bereich gelegen ist. Dadurch ist sichergestellt, dass im Normalzustand der Betriebszustand des Schmiermittels erfasst werden kann.

In dem Anwendungsfall, in dem für den Betriebszustand des Gehäuses mit dem erfindungsgemäßen Gehäuseteil 10 da Gehäuse nur teilweise mit Schmiermittel gefüllt ist, kann es sich bei der Öffnung, in die eine erfindungsgemäße Verschlussvorrichtung eingesetzt ist, insbesondere um eine Schmiermittelauslass-Öffnung handeln, die bei einer bestimmungsgemäßen Orientierung des Gehäuses unterhalb des Oberflächenspiegels S1 des im Gehäuse für den Betrieb befindlichen Schmiermittels S gelegen ist.

Bei einem speziellen Anwendungsbeispiel der Erfindung, bei der das Gehäuseteil ein Teil eines Gehäuses oder das Gehäuse für einen Leistungs-Übertragungsmechanismus 11 und insbesondere einen Rotationsaktuator zum Verstellen einer aerodynamischen Klappe eines Flugzeugs ist (Figur 2), kann insbesondere vorgesehen sein, dass das Gehäuse mit dem erfindungsgemäßen Gehäuseteil nur zwischen 40 % und 80 % des Gehäusevolumens mit Schmiermittel gefüllt ist. Der Einbau des Gehäuses mit dem erfindungsgemäßen Gehäuseteil und die Lage der Öffnung an dem Gehäuse ist derart vorgesehen, dass die Öffnung mit der erfindungsgemäßen Verschlussvorrichtung unterhalb des Oberflächenspiegels S1 des im Gehäuse für den Betrieb befindlichen Schmiermittels S gelegen ist.

Das in der Figur 2 dargestellte Ausführungsbeispiel eines erfindungsgemäßen Gehäuses 10 ist zur Aufnahme eines Rotationsaktuators R oder Drehaktuators als Leistungs-Übertragungsmechanismus 11 vorgesehen und ist aus einem drehbaren Gehäuseteil 30 und einem zweiten Gehäuseteil gebildet, das als Grundkörper 32 oder Flanschkörper ausgebildet ist, wobei das Gehäuseteil 30 auf dem Grundkörper 32 drehbar angeordnet ist. Der Grundkörper 32 ist zur Befestigung an einem Strukturbauteil vorgesehen und weist zu diesem Zweck zwei Flanschbohrungen 32a, 32b auf. Das drehbare Gehäuseteil 30 bildet auf seiner Innenseite 30a einen Innenraum A aus der von der diesem zugewandten Innenseite des Grundkörpers 32 teilweise begrenzt wird. In der Figur 1 ist die Innenwand 30a des drehbaren Gehäuseteils 30 als Strichlinie dargestellt. Im Innenraum A ist ein Übertragungsmechanismus 11 (nicht dargestellt) eingebaut, mit dem eine mit einer Eingangswelle auf den Übertragungsmechanismus 11 übertragene Eingangsleistung durch den Übertragungsmechanismus 11 auf eine Ausgangswelle (nicht dargestellt) übertragen wird. Der Übertragungsmechanismus 11 kann als ein Getriebe und insbesondere als Zahnrad-Getriebe realisiert sein. Die Ausgangswelle ist mit einem Stellhebel 33 gekoppelt, der über eine Kopplungsvorrichtung 34 mit einer Übertragungsstange gekoppelt ist.

Der dargestellte Rotationsaktuator R kann insbesondere zur Verstellung einer aerodynamischen Klappe eines Flugzeugs verwendet werden. Die aerodynamische Klappe kann insbesondere eine Hinterkantenklappe eines Hauptflügels des Flugzeugs sein. Bei dieser Anwendung ist der Grundkörper 32 an einem Strukturbauteil des Hauptflügels befestigt und die Übertragungsstange an die aerodynamischen Klappe bzw. die Hinterkantenklappe gekoppelt.

Bei dem Gehäuseteil 10 nach der Erfindung kann es sich insbesondere um ein Gehäuseteil handeln, das relativ zu einem anderen Gehäuseteil bewegbar oder drehbar ist und eine dynamische Dichtung zwischen den Gehäuseteilen gelegen ist. Unter "dynamische Dichtung" wird in diesem Zusammenhang eine Dichtung verstanden, die zwischen sich relativ zueinander bewegbaren Teilen gelegen ist und die zwar einen durch die Teile gebildeten Innenraum in Bezug auf in diesem befindlichem Schmierfluid abdichtet, jedoch den Innenraum nicht in Bezug auf Luft vollständig abdichtet. Bei dem Gehäuseteil 10 nach der Erfindung kann es sich auch um ein Gehäuseteil 10 mit einer Ausnehmung handeln, durch die sich ein drehendes Teil wie z.B. eine Eingangswelle oder generell ein sich gegenüber dem Gehäuseteil bewegbares Teil hindurch ragt, wobei zwischen dem sich gegenüber dem Gehäuseteil bewegenden Teil und dem Gehäuseteil 10 eine dynamische Dichtung gelegen ist. Das Gehäuseteil weist in diesem Fall also eine Aufnahme zur Lagerung einer dynamischen Dichtung auf.

Das erfindungsgemäße Gehäuseteil kann, jedoch muss nicht ausschließlich als Schmiermittelbehälter ausgebildet und nicht ausschließlich für den Zweck der Schmiermittelaufnahme vorgesehen sein. Wie in der Figur 1 ersichtlich, kann das Gehäuseteil 10 auch die Funktion einer Abdeckung sowie z.B. auch die Funktion eines Stellelements, im Ausführungsbeispiel nach der Figur 1 als Stellhebel 33 realisiert, haben. Das Gehäuseteil 10 ist jedoch Teil eines Gehäuses oder das Gehäuse, in dem sich für den bestimmungsgemäßen Betrieb Schmiermittel befindet.

Bei dem dargestellten Rotationsaktuator R zum Verstellen einer aerodynamischen Klappe eines Flugzeugs ist (Figur 2) ist insbesondere vorgesehen, dass das Gehäuse mit dem erfindungsgemäßen Gehäuseteil nur zu maximal 80 % des Gehäusevolumens mit Schmiermittel gefüllt ist. Die in der Figur 2 dargestellte Orientierung des Grundkörpers 32 ist diejenige Orientierung, in der dieser in ein Strukturbauteil eines Flugzeugs eingebaut oder an diesem befestigt ist. In der Figur 2 ist ein XY-Koordinatensystem eingetragen, wobei die Y-Richtung die Schwerkraftrichtung angibt. Das Gehäuseteil 30 ist in einer Stellung gezeigt, in der die Übertragungselement 35 die an dieser angekoppelten Klappe in einem ausgefahrenen Zustand hält. Das Gehäuseteil 30 wird also aus der Blickrichtung eines Betrachters der Figur 2 gesehen entgegen den Uhrzeigersinn gedreht, wenn der Rotationsaktuator R das Übertragungselement 35 in eine eingefahrene Stellung bringt.

Das in der Figur 2 dargestellte Ausführungsbeispiel des Rotationsaktuators R weist in dem Grundkörper 32 eine Einlass-Öffnung 41 zum Einführen von Schmiermittel und eine Auslassöffnung 42 zum Auslassen des Schmiermittels auf. Auch weist das Gehäuseteil 30 des Rotationsaktuators R nach der Figur 2 ebenfalls eine Einlass-Öffnung 43 zum Einführen von Schmiermittel und eine Auslassöffnung 44 zum Auslassen des Schmiermittels auf. Nach einer alternativen Ausführungsbeispiel kann an dem Rotationsaktuators R nach der Figur 2 auch nur jeweils eine Einlassöffnung und nur eine Auslassöffnung vorgesehen sein, wobei in diesem Fall die Einlassöffnung und die Auslassöffnung jeweils an dem Gründkörper 32 und/oder an dem drehbaren Gehäuseteil 30 angeordnet sein können.

Da der Einbau des Gehäuses mit dem erfindungsgemäßen Gehäuseteil und die Lage der Öffnung mit einer Verschlussvorrichtung nach der Erfindung an dem Gehäuse derart vorgesehen ist, dass die Öffnung mit der erfindungsgemäßen Verschlussvorrichtung unterhalb des Oberflächenspiegels S1 des im Gehäuse für den Betrieb befindlichen Schmiermittels S gelegen ist, sind in dem dargestellten Ausführungsbeispiel die Auslassöffnungen 42, 44 jeweils mit einer Verschlussvorrichtung nach der Erfindung versehen. Alternativ kann auch nur eine der Auslassöffnungen, also die Auslassöffnung am Grundkörper 32 oder die Auslassöffnung am drehbaren Gehäuseteil 30, mit der erfindungsgemäßen Verschlussvorrichtung versehen sein. Die weiteren Öffnungen können jeweils eine Verschlussvorrichtung ohne Sensorvorrichtung eingesetzt sein, um diese zu verschließen.

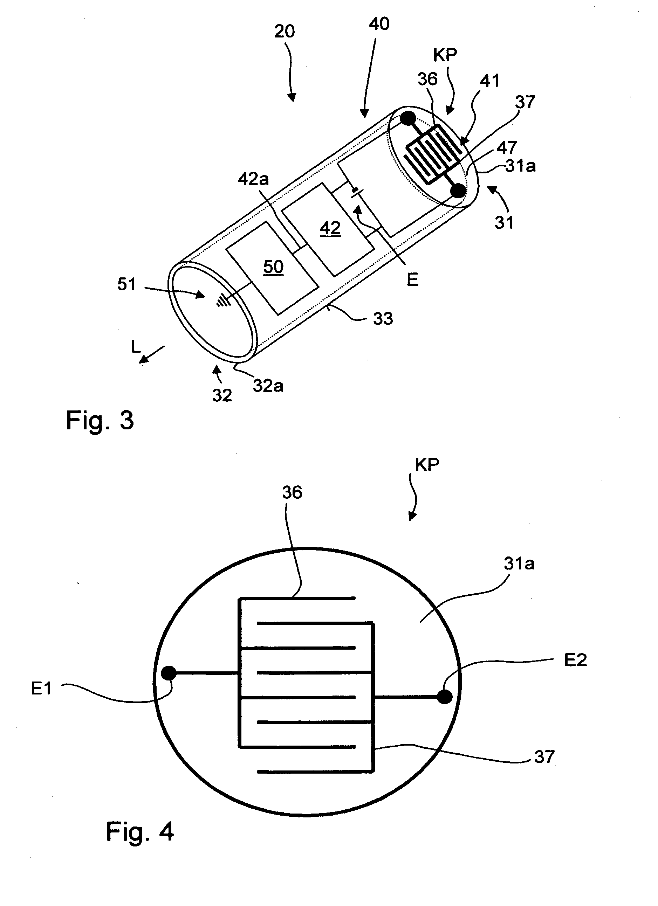

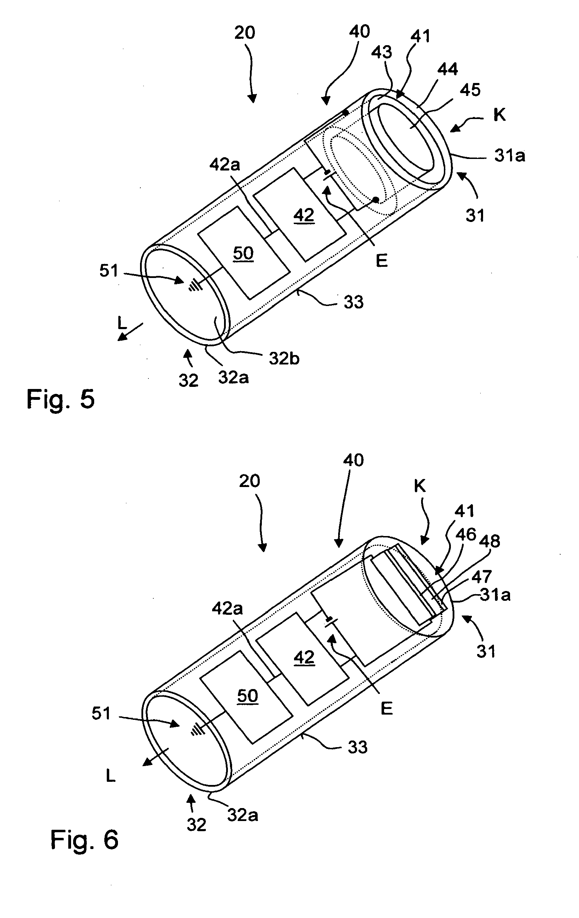

In den Figuren 3, 5 und 6 sind zwei Ausführungsformen der Verschlussvorrichtung 30 dargestellt, wobei Figuren 5 und 6 keine Ausführungsforms der Erfindung darstellen, die generell einen ersten Endabschnitt 31 mit einer ersten Endfläche 31a, einen entgegen gesetzt zu diesem gelegenen zweiten Endabschnitt 32 mit einer zweiten Endfläche 32a und eine sich entlang der Längsrichtung L erstreckende Längsseite 33 oder Außenseite aufweist. In der Verschlussvorrichtung 20 ist eine Energieversorgungsvorrichtung E, eine Sensorvorrichtung 40 mit einem Sensor 41 und einer Signalverarbeitungsvorrichtung 42 sowie ein mit der Signalverarbeitungsvorrichtung 42 über eine Verbindungsleitung 42a funktional in Verbindung stehender Transceiver 50 oder ein Senderempfänger angeordnet, der eine Signalübertragungs-Vorrichtung 51 zur Übertragung von Ausgangssignalen, die von der Signalverarbeitungsvorrichtung 42 erzeugt worden sind, an eine externe Empfangsvorrichtung und zum Empfang von Ansteuerungssignalen von einer externen Empfangsvorrichtung.

Nach einem Ausführungsbeispiel ist die erfindungsgemäße Verschlussvorrichtung 20 als Schraube und zum Einsetzen in eine Gewindeaufnahme einer Öffnungen, also der Einlassöffnung oder der Auslassöffnung, ausgebildet sein. Hierzu ist die Längsseite 33 mit einem Außengewinde (nicht gezeigt) und die jeweilige Öffnung als Innengewinder (nicht gezeigt) ausgeführt. Alternativ kann die Verschlussvorrichtung nach der Erfindung als Bolzen mit einem Verankerungselement zum Verschließen der jeweiligen Öffnung, also der Einlass- oder Auslassöffnung mit dem Bolzen ausgebildet sein. Auch kann die erfindungsgemäße Verschlussvorrichtung als anderes lösbares Verschlusselement gestaltet sein.

Der Sensor 41 der Sensorvorrichtung 40 ist erfindungsgemäß generell ein Sensor, der durch Eintauchen in das Schmiermittelbad bei der in das Gehäuseteil eingesetzten Verschlussvorrichtung 20 Betriebsparameter des Schmiermittels erfassen kann, die zumindest den Wassergehalt des Schmiermittels und ggf. z.B. die Temperatur und/oder den Druck des Schmiermittels erfassen kann, indem die erste Endfläche 31 a des ersten Endabschnitts 31 mit dem Schmiermittel in Kontakt ist. Die erste Endfläche 31a ist dabei Bestandteil des Sensors 41 und zur Erfassung des jeweiligen Betriebsparameters ausgeführt.

Zur Erfassung des Wassergehalts im Schmiermittel ist der Sensor als Kondensator ausgeführt. Dabei weist die Endfläche 31a zwei Kondensator-Teile auf, um die Wirkung der dielektrischen Eigenschaften des Schmiermittels nach dem Impedanz-/Kapazitäts-Messprinzip zu erfassen. Genauer ist die erste Endfläche 31 a bzw. die Sensorvorrichtung 40 aus einem Planar-Kondensator KP mit zwei Kondensator-Flächen 36, 37 gebildet, die flächig oder gabelförmig ineinander greifen, um die Größe der einander zugewandten Randlinien oder Randflächen zu maximieren (Figur 3). Die nach außen gerichteten Planarkondensator-Teilflächen der Planarkondensator-Elektroden oder Planarkondensator-Teilplatten 36, 37 erstrecken sich in der Endfläche 31a. Zwischen den Planarkondensator-Elektroden 36, 37 kann eine Nut vorgesehen oder ein Isolationsmaterial gelegen sein. Bei einem weiteren Ausführungsbeispiel können die Kondensator-Teile zwei Antennen sein, die von der ersten Endfläche 31 a wegragen (in den Figuren nicht dargestellt).

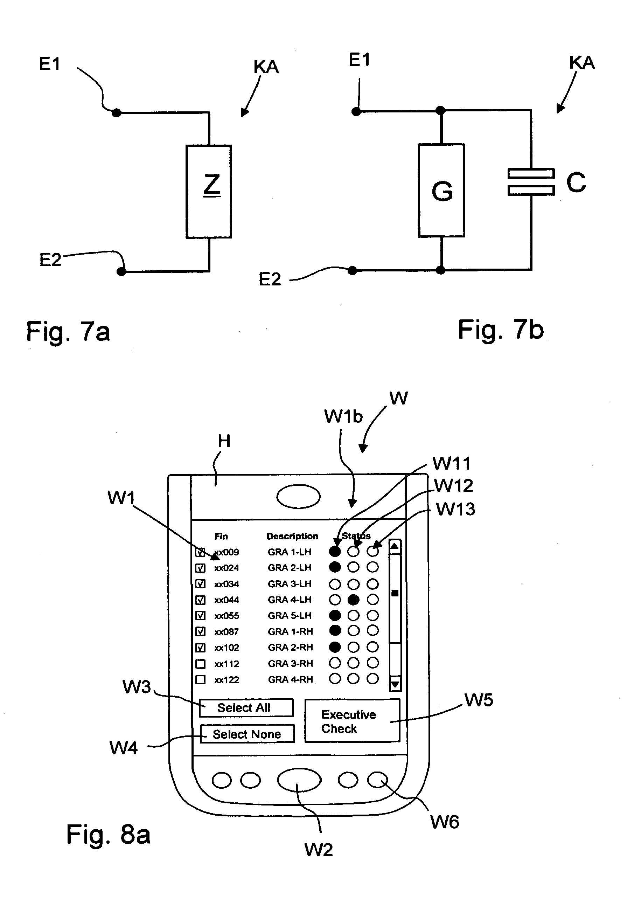

Mit der Energieversorgungsvorrichtung E wird an die Kondensator-Teile oder die Kondensator-Platten des jeweils vorgesehenen Kondensators oder der KondensatorAnordnung KA Wechselspannung angelegt. In der Figur 4 sind zur Darstellung der Verbindung der Energieversorgungsvorrichtung E mit den jeweiligen KondensatorTeilen - in dem dargestellten Beispiel mit den Planarkondensator-Elektroden 36, 37 - zur deutlicheren Darstellung schematisch Kontaktstellen E1 und E2 eingezeichnet, die bei einer Realisierung der dargestellten Vorrichtungen nicht vorhanden sein müssen. In einer Messfunktion wird aus der angelegten Wechselspannung eine Impedanz ermittelt, wie dies in der Figur 7a mit einem elektrischen Ersatzschaltbild schematisch dargestellt ist. Die Impedanz eines Kondensators wird durch seine Geometrie und die elektrischen Eigenschaften des sich zwischen den Köndensatorplatten befindlichen Dielektrikums bestimmt. Allgemein kann die Impedanz als komplexer Widerstand betrachtet werden. Alternativ oder zusätzlich kann vorgesehen sein, dass mit einem nach der Erfindung vorgesehenen Kondensator bei einer angelegten Wechselspannung eine Amplitude und/oder eine Phasenverschiebung und/oder eine Dämpfung gemessen wird, die jeweils die gemessene Wechselspannung in Bezug auf die angelegte Wechselspannung aufweist, um daraus z.B. in der Sensorvorrichtung oder mittels der Messfunktion eine relative Dieelektrizitätskonstante epsilon-r (εᵣ) zu ermitteln. In der Figur 7b ist ein Ersatzschaltbild einer technischen Realisierung einer Ausführungsform eines nach der Erfindung vorgesehenen KondensatorAnordnung KA mit einem Kondensator C und einem parallel zu diesem geschalteten Widerstand G dargestellt, mit der die genannten Messsignale bzw. Messgrößen ermittelt werden können. Im Kondensator C wird die gespeicherte elektrische Energie beschrieben, im Parallelleitwert G die elektrische Leitfähigkeit.

Nach einem Beispiel kann der Kondensator K durch das Ausbilden einer in der Endfläche 31a umlaufenden Nut 43 gebildet sein, so dass der Endabschnitt 31 aus einem am äußeren Rand der ersten Endfläche 31 a umlaufenden Kondensator-Ring 44 und einem innerhalb desselben ausgebildeten Kondensator-Stempel 45 gebildet ist (Figur 5). Die einander zugewandten Oberflächen des Kondensator-Rings 44 und des Kondensator-Stempels 45, also die Innenfläche des Kondensator-Rings 44 und die Außenfläche des Kondensator-Stempels 45 bilden die einander gegenüber liegenden Kondensatorflächen, zwischen denen sich das Schmiermittel befindet, für das mit dem Kondensator K der Wassergehalt gemessen werden soll. nach einem alternativen Beispiel kann der Endabschnitt 31 bzw. der Sensor 41 auch als Platten-kondensator mit zwei z.B. parallel zueinander verlaufenden und von der ersten Endfläche 31a Kondensatorplatten 46, 47 und einem zwischen diesen gelegenen Zwischenraum 48 nach der Figur 6 gebildet sein. Bei diesen beiden Beispielen handelt es sich nicht um Ausführungsformen der Erfindung, sondern um Beispiele, die das Verständnis der Erfindung erleichtern.

Nach einem Ausführungsbeispiel der Erfindung ist der Sensor 41 derart ausgeführt, dass dieser zumindest einen Bereich zwischen 0% und 40% relativer Feuchte erfassen kann. Der Vorteil der erfindungsgemäß vorgesehenen Sensoren und insbesondere der Verwendung von Kondensatoren zur Feuchtigkeitsermittlung ist, dass derartige Messbereiche und erforderliche Messgenauigkeiten erreicht werden können.

Insbesondere durch die Ausführung des Sensor als Kondensator-Sensor mit strukturell realisierten Kondensator-Platten wird als Dielektrikum der Schmierstoff direkt verwendet. Das Messsignal wird maßgeblich durch die relative Dielektrizitätszahl bestimmt. Schmierstoffe weisen eine relative Dielektrizitätskonstante εᵣ (epsilon-r) □von ca. 3-5 auf. Wasser weist hingegen durch die atomare Wasserstoffbrückenbildung eine relative Dielektrizitätskonstante εᵣ (epsilon-r) von ca. 80 auf. Dadurch lässt sich Wasser sehr gut vom Schmierstoff unterscheiden und der Wasseranteil in Schmierstoffen sehr leicht erfassen und nachweisen.

Die Sensorvorrichtung 40 kann insbesondere eine mit dem Sensor 41 funktional verbundene Signalverarbeitungs-Vorrichtung 42 aufweisen, die die elektrischen Signale des Sensors erfasst. Die Signalverarbeitungsvorrichtung 42 kann auch eine Vergleichsfunktion aufweisen, die die erfassten und je nach dem Sensor Sensorsignale, die einem Feuchtigkeitsgehalt, einem Druck oder eine Temperatur entsprechen, mit einem Grenzwert oder mit mehreren Grenzwerten vergleicht und ein Ausgabesignal erzeugt, das einem Unterschreiten eines Grenzwerts, einem Überschreiten eines Grenzwertes oder einem zwischen zwei Grenzwerten liegen des Sensorsignals entspricht und dieses anzeigt.

Die Sensorvorrichtung 40 und insbesondere die Signalverarbeitungsvorrichtung 42 kann mit dem Transceiver 50 zum Empfang der Sensorsignale und/oder der Ausgabesignale von der Sensorvorrichtung und zur Signalübertragung der Sensorsignale an eine externe Empfangseinheit über eine Verbindungsleitung 42a funktional verbunden sein. Der Transceiver 50 ist zu diesem Zweck mit einer Signalübertragungs-Vorrichtung 51 verbunden. Nach den in den Figuren 3, 5 und 6 dargestellten Ausführungsbeispielen ist die Signalübertragungs-Vorrichtung 51 als Antenne zur Übertragung von Signalen und Informationen über Funk und somit kabellos an einem externe Empfangseinheit, an die wiederum eine Signalverarbeitungs-Vorrichtung zur weiteren Verarbeitung und insbesondere zur Bewertung des Erfordernisses von Wartungsmaßnahmen gekoppelt ist. Alternativ dazu kann die Signalübertragungs-Vorrichtung 51 eine Draht- oder Kabelverbindung zu der externen Empfangseinheit realisiert sein.

Die externe Empfangseinheit ist eine Einheit außerhalb der Verschlussvorrichtung und insbesondere eine Einheit außerhalb des Gehäuses mit dem erfindungsgemäßen Gehäuseteil 20. Dabei kann die externe Empfangseinheit Teil eines Wartungs-Handgeräts H sein.

Durch diese kabellose Ausführung der Signalverbindung zwischen dem Transceiver 50 und einer externen Verarbeitungsvorrichtung oder externen Wartungsvorrichtung ergibt sich eine besonders einfache und kompakte Bauweise, da eine zusätzliche Verkabelung eingespart wird und weil dann, wenn ein Sensor bzw. ein Transceiver ausfallen sollte, es ausreichend wäre, einfach nur die Verschlussvorrichtung 20 mit dem defekten Sensor/Transceiver durch eine Verschlussvorrichtung 20 mit funktionsfähigem Sensor/Tansceiver zu ersetzen, ohne dass noch weitere Steckverbindungen zu lösen oder zu befestigen wären.

Der Transceiver 50 kann in einer Ausnehmung eines zweiten als Kopfende ausgebildeten und entgegen gesetzt zu dem ersten Endabschnitt 31 gelegenen Endabschnitts 32 der Verschlussvorrichtung 20 angeordnet sein, wobei der zweite Endabschnitt 32 der Verschlussvorrichtung eine Außenseite aufweist 32a, die in dem das Gehäuseteil 10, 30 schließenden Zustand der Verschlussvorrichtung eine Außenseite des Gehäuseteils 10, 30 bildet, und wobei ein Innenbereich der Außenseite des zweiten Endabschnitts der Verschlussvorrichtung zur Funkübertragung der Sensorsignale an eine externe Empfangseinheit eine Öffnung 32b der Ausnehmung aufweist. Dabei kann in die Öffnung 32b eine Kunststoffschicht eingesetzt sein, die für die Übertragungswellen per Funk durchlässig ist. Die Außenseite des zweiten Endabschnitts 32 der Verschlussvorrichtung mit der Öffnung kann auch vollständig oder teilweise mit einer Kunststoffschicht überdeckt sein.

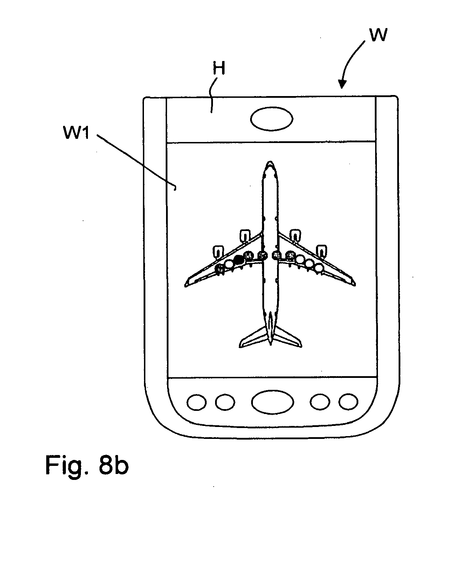

Besonders vorteilhaft ist es, wenn erfindungsgemäße Gehäuseteile mit in einem Schmiermittel gelagerten beweglichen Teilen durch ein kabelloses Diagnosesystem überwacht werden, wie es beispielartig anhand der Figuren 8a und 8b illustriert ist. Dabei zeigen die Figuren 8a und 8b jeweils ein Handgerät als Ausführungsbeispiel einer externen Verarbeitungsvorrichtung für ein kabelloses Diagnosesystem, in dem eine Verarbeitungseinheit zum Empfang und zur Verarbeitung von Sensorsignalen, die von den Sensoren 41 in einem erfindungsgemäßen Gehäuseteil 10 erfasst und von den zugehörigen Transceivern ausgestrahlt werden, und eine Anzeigeeinrichtung integriert sind. Auf der Anzeigeeinrichtung ist in der Figur 8b am Beispiel eines Flugzeugs der Einsatz mehrerer erfindungsgemäßer Gehäuseteile 10 visualisiert, die an bestimmten, jeweils durch Kreise veranschaulichten Positionen an den Tragflächen des Flugzeugs eingebaut sind.

Die in den Verschlussvorrichtungen 20 von am Flugzeug eingesetzten erfindungsgemäßen Gehäuseteilen sich befindenden Sensoren geben dabei Sensorsignale an die ebenfalls in den Verschlussvorrichtungen 20 der Gehäuseteile integrierten Transceiver 50 ab, die diese wiederum an die externe Verarbeitungseinheit übermitteln. Die Verarbeitungseinheit erkennt, von welchem Gehäuseteil ein Sensorsignal kommt und zeigt dies auf der Anzeigeeinrichtung des Handgeräts an. Weiterhin wertet die Verarbeitungseinheit die eintreffenden Sensorsignale aus, um daraus Rückschlüsse über den Zustand des Schmiermittels in den jeweiligen Gehäuseteilen zu ziehen und Diagnoseinformationen, wie z.B. in Fig. 6a gezeigt, anzuzeigen.

Die separate Wartungsvorrichtung W kann insbesondere einen Transceiver zum Empfang von Sensorsignalen von dem der Sensorvorrichtung 40 zugeordneten Transceiver 50 aufweisen, wobei die Wartungsvorrichtung eine Verarbeitungsfunktion zur Ermittlung eines Wertes für den Betriebszustand eines in dem Gehäuseteil befindlichen Schmiermittels aus dem Sensorsignal aufweist. Die separate Wartungsvorrichtung W kann auch, z.B. aufgrund einer entsprechenden Eingabe an derselben ein Steuerungskommando erzeugen, das über den Transceiver 50 der Verschlussvorrichtung 20 an die Sensorvorrichtung 40 übermittelt wird, das die Sensorvorrichtung 40 zur Durchführung einer Messung aktiviert.

Die Wartungsvorrichtung W kann eine Anzeigevorrichtung W1 zur Anzeige der Sensorgrößen und/oder von ermittelten Wartungsinformationen aufweisen. Ein Ausführungsbeispiel eines mit der Anzeigevorrichtung darstellbaren Anzeigeformats kann aufweisen: einen Bereich W1a mit einer Definition oder Angabe zumindest eines jeweils abgefragten oder angezeigten Sensors und/oder zumindest einer Komponenten, in der der jeweilige Sensor eingebaut ist, dargestellt sind, und einen Bereich W1b, in dem der technische Status des jeweils angezeigten Sensors gezeigt sind. Das Anzeigeformat kann, wie auch in der Figur 8a schematisch gezeigt ist, derart gestaltet sein, dass zeilenförmig verschiedene Sensoren 41 oder Sensorvorrichtungen 40 oder Komponenten wie z.B. Aktuatoren, in denen diese jeweils eingebaut ist, aufgelistet sind und z.B. rechts seitlich davon drei Bewertungsspalten W11, W12, W13 z.B. in Form von drei in derselben Zeile jeweils nebeneinander angeordnete markierbare Anzeigeoptionen und z.B. Leuchtanzeigen vorgesehen sind, wie dies nur andeutungsweise in der Figur 8a dargestellt ist. Dabei kann das Markieren oder Nicht-Markieren von Feldern innerhalb jeweils einer Zeile also für jeweils eine Verschlussvorrichtung oder ein Gerät mit einer oder mehreren Verschlussvorrichtungen mit folgenden Bedeutungen hinsichtlich erforderlicher Wartungsmaßnahmen folgt vorgesehen sein:
▪ das Markieren eines Feldes der ersten Spalte: kein Austausch des Schmiermittels erforderlich, da der gemessene Wassergehalt oder die gemessene relative Feuchte in dem Schmiermittelbehälter unter einem ersten vorgegebenen Grenzwert liegt;
▪ das Markieren eines Feldes der ersten Spalte und zusätzlich das Markieren eines Feldes der dritten Spalte: kein Austausch des Schmiermittels erforderlich, da der gemessene Wassergehalt oder die gemessene relative Feuchte in dem Schmiermittelbehälter zwar über dem ersten vorgegebenen Grenzwert, jedoch unter einem zweiten vorgegebenen Grenzwert liegt, allerdings ist die Notwendigkeit des Austausches des Schmiermittels zu überwachen, weil der Austausch des Schmiermittels in einem absehbaren Zeitraum erforderlich werden kann;
▪ das Markieren eines Feldes der zweiten Spalte: es liegt keine ausreichend gute Signalqualität bei der Übertragung von Signalen oder Daten zwischen der Wartungsvorrichtung und den betreffenden und insbesondere jeweils angewählten Sensorvorrichtungen 40 oder Komponenten, in denen die jeweils angewählte Sensorvorrichtung 40 eingebaut ist;
▪ das Markieren eines Feldes der dritten Spalte: ein Austausch des Schmiermittels ist erforderlich, da der gemessene Wassergehalt oder die gemessene relative Feuchte in dem Schmiermittelbehälter zwar über dem zweiten vorgegebenen Grenzwert liegt;
▪ das Markieren keines der Felder der Spalten einer Zeile: es wurde mittels der Wartungsvorrichtung W keine Abfrage durchgeführt.

In der Darstellung der Figur 8a sind einige der kreisförmigen Felder grau ausgefüllt. Dadurch soll schematisch beispielartig die an verschiedenen Sensorvorrichtungen erfassten Schmiermittelzustände angezeigt werden, je nach dem welche Felder einer zu einer Sensorvorrichtung jeweils gehörenden Zeile markiert, d.h. in der Figur 8a grau ausgefüllt sind.

Dabei kann insbesondere vorgesehen sein, dass der erste Grenzwert in dem Bereich zwischen 6 % und 10 % und der zweite Grenzwert in dem Bereich zwischen 12 % und 16 % liegt.

Die Anordnung von Feldern kann so realisiert sein, dass in dem Anzeigeformat in jeder Spalte einer Zeile jeweils ein markierbares Feld angeordnet ist. Das jeweilige Feld z.B. kreisförmig gebildet sein. Das einer Zeile und einer Spalte jeweils zugeordnete Feld kann als ein Bereich innerhalb eines einheitlichen Anzeigebereichs z.B. eines LCD-Anzeigebereichs oder als Lampe oder separate Leuchtanzeige realisiert sein.

Zusätzlich kann vorgesehen sein, dass die Felder jeweils unterschiedlicher Spalten in unterschiedlichen Farben dargestellt sind oder unterschiedliche Farben annehmen und/oder in unterschiedlichen Farben aufleuchten, wenn diese z.B. aufgrund einer Abfrage markiert oder hervorgehoben werden sollen. Z.B. kann vorgesehen sein, dass die Felder der ersten Spalte in grün, die Felder der zweiten Spalte in gelb und die Felder der dritten Spalte in rote Farbe annehmen, wenn diese markiert oder angezeigt werden sollen.

Zur entsprechenden Realisierung dieser Kontrollfunktionen in einer der Ausführungsbeispiele nach der Erfindung kann die Sensorvorrichtung eine Funktion aufweisen, die eine Vergleichsfunktion aufweist, in der zwei Grenzwerte abgespeichert oder mit der zwei Grenzwerte vorgegeben sind. Der erste Grenzwert kann ein zwischen 6 % und 10 % relativer Feuchte definierter Wert und der zweite Grenzwert kann ein zwischen 12 % und 16% relativer Feuchte definierter Wert sein. Z.B. kann der erste Grenzwert gleich 8% und der zweite Grenzwert gleich 12 % sein. Bei diesem Ausführungsbeispiel kann die Sensorvorrichtung und/oder die Wartungsvorrichtung W derart ausgeführt sein, dass das erfasste Messsignal der Vergleichsfunktion zugeführt wird und dass die Vergleichfunktion ermittelt, in welchen der durch die zwei Grenzwerte abgegrenzten Bereiche der jeweils erfasste Signalwert gelegen ist (entweder unterhalb des ersten Grenzwerts oder zwischen den beiden Grenzwerten oder oberhalb des zweiten Grenzwertes), so dass aufgrund der Identifizierung eines durch die Grenzwerte definierten Bereichs eine Markierung des Feldes in der entsprechenden Spalte des Anzeigenformats erfolgt. Dadurch kann für die drei Bereiche die Dringlichkeit einer Wartungsmaßnahme nach einer der hiermit beschriebenen Arten bewertet und angezeigt werden.

Es kann somit ein Diagnoseverfahren zur Überwachung eines Schmiermittelmediums mittels der Sensorvorrichtung und einer dieser zugeordneten Wartungsvorrichtung durchgeführt werden. Dabei wird ein durch eine Sensorvorrichtung erfasstes Messsignal einer Vergleichsfunktion zugeführt, wobei die Vergleichsfunktion das jeweils erfasste Messsignal mit zwei Grenzwerten vergleicht und aufgrund dieses Vergleichs identifiziert, ob das jeweils erfasste Signalwert in einem ersten Bereich unterhalb eines ersten Grenzwerts oder in einem zweiten Bereich zwischen dem ersten und einem zweiten Grenzwert, der größer als der erste Grenzwert ist, oder in einem dritten Bereich oberhalb des zweiten Grenzwertes gelegen ist. Aufgrund der Identifizierung eines Bereichs für den jeweils erfassten Signalwert erfolgt eine Markierung eines von drei Feldern, die jeweils einem der Bereich zugeordnet sind, in einem Anzeigeformat einer Anzeigevorrichtung erfolgt.

Bei dem Diagnoseverfahren kann vorgesehen sein, dass der erste Grenzwert ein Wert für die relative Feuchte ist, der zwischen 6 % und 10 % gelegen ist, und dass der zweite Grenzwert ein Wert für die relative Feuchte ist, der zwischen 12 % und 16% gelegen ist.

Alternativ oder zusätzlich kann bei dem Diagnoseverfahren vorgesehen sein, dass nach dem Anzeigeformat einer Anzeigevorrichtung für jeweils eine Sensorvorrichtung, mit der das Messsignal erfasst worden ist, dieser Sensorvorrichtung jeweils drei Felder zugeordnet sind, von denen jeweils ein Feld in einer von drei Spalten des Anzeigeformats angeordnet sind, wobei bedeuten:
▪ das Markieren eines Feldes der ersten Spalte, dass das erfasste Messsignal unter dem ersten vorgegebenen Grenzwert liegt;
▪ das Markieren eines Feldes der ersten Spalte und zusätzlich das Markieren eines Feldes der dritten Spalte, dass das erfasste Messsignal zwischen dem ersten vorgegebenen Grenzwert und dem zweiten vorgegebenen Grenzwert liegt;
▪ das Markieren eines Feldes der zweiten Spalte, dass keine ausreichend gute Signalqualität bei der Übertragung von Signalen oder Daten zwischen der Wartungsvorrichtung und den betreffenden und insbesondere jeweils angewählten Sensorvorrichtung oder den diesen jeweils zugeordneten Transceivern vorliegt;
▪ das Markieren eines Feldes der dritten Spalte, dass das erfasste Messsignal über dem zweiten vorgegebenen Grenzwert liegt;
▪ das Markieren keines der Felder der Spalten einer Zeile, dass mittels der Wartungsvorrichtung W keine Abfrage durchgeführt worden ist.

Das Markieren von Feldern kann mit einem Multifunktionsschalter W2 erfolgen. Weiterhin kann die Wartungsvorrichtung W oder das Handgerät H einen Schalter W3 zum Anwählen aller ansteuerbaren Sensoren oder Sensorvorrichtungen aufweisen ("select all"). Weiterhin kann ein Schalter W4 vorgesehen sein, mit dem die Auswahl von Sensoren oder Sensorvorrichtungen ausgeschlossen werden kann ("select none"). Weiterhin kann ein Schalter W5 vorgesehen sein, mit dem bei einem oder mehreren von jeweils ausgewählten Sensoren oder Sensorvorrichtungen von der Wartungsvorrichtung W ein Aktivierungssignal an diese Sensoren oder Sensorvorrichtungen gesendet werden kann, so dass mit diesen eine Messung durchgeführt wird und/oder von diesen ein dort abgespeicherter Messwert oder Signalwert ausgelesen und an die Sensoren oder Sensorvorrichtungen geschickt wird.

Auch kann vorgesehen sein, dass z.B. mit einem weiteren Schalter W6 die Einbauorte Sensoren oder Sensorvorrichtungen an dem System oder Fahrzeug, für das die Wartungsvorrichtung eingesetzt wird, angezeigt werden, In der Figur 8b ist ein solche Anzeige für den Fall dargestellt, dass das Wartungssystem und die erfindungsgemäße Sensorvorrichtung für ein Flugzeug angewendet wird. Bei der Ausführungsform, bei der an einer oder mehreren Sensorvorrichtungen und insbesondere Verschlussvorrichtungen 20 ein Satelliten-Positionsermittlungssensor integriert ist, kann die Zuordnung der jeweiligen Sensorvorrichtung über die ermittelte Position der jeweiligen Sensorvorrichtung erfolgen. Dies kann zusätzlich oder alternativ zu der Übermittlung einer Adresskennung der jeweils nach einem Sensorwert abgefragten Sensorvorrichtung erfolgen.

Dementsprechend kann die Wartungsvorrichtung W ein Funktionsmodul aufweisen, mit dem aus einem Sensorsignal ein Wert für einen Betriebszustand eines in dem Gehäuseteil befindlichen Schmiermittels ermittelt werden kann, mit dem eine für eine Wartungsaufgabe notwendige Wartungsinformation an eine Anzeigeeinrichtung übermittelt und mit der Anzeigeeinrichtung angezeigt werden kann.

Besonders vorteilhaft ist dabei, wenn das Diagnosesystem Sensorsignale, die von verschiedenen in ein und demselben Gehäuseteil (z.B. in der Einlassöffnung und in der Auslassöffnung) angebrachten Sensoren an die Verarbeitungseinheit übermittelt, und dort miteinander vergleicht, wobei als Ergebnis des Vergleichs ein Vergleichsfunktionswert gebildet und der weiteren Verarbeitung zugrunde gelegt wird. So könnte von zwei von ein und demselben Gehäuseteil kommenden Sensorsignalen z.B. ein Mittelwert gebildet werden oder es würde grundsätzlich das kleinere der beiden Sensorsignale verworfen werden. Letzteres wäre z.B. eine besonders einfache und effektive Vorgehensweise für Fälle, wo einer der beiden Sensoren in der Ein- bzw. Auslassöffnung des Gehäuseteils defekt ist und ein viel zu kleines oder gar kein Sensorsignal liefert.

Weiterhin ist es vorteilhaft, wenn die externe Verarbeitungseinheit die von einer Sensorbaugruppe empfangenen Signale mit vorgegebenen Grenzwerten für diese Signale vergleicht und auf der Anzeige visualisiert, ob das empfangene Signal innerhalb vorgegebener Grenzwerte für das Signal liegt (Figur 8a).

Für den Fall, dass ein empfangenes Sensorsignal außerhalb des Bereichs der vorgegebenen Grenzwerte liegt, kann die Verarbeitungseinheit ein Warnsignal, insbesondere ein akustisches oder optisches Warnsignal, ausgeben.

Für den Fachmann ist es ohne weiteres ersichtlich, dass das Prinzip des in den Figuren 8a und 8b beispielsweise veranschaulichten Diagnosesystems mit der Wartungsvorrichtung H und der damit durchgeführten Diagnoseverfahren auch zur Unterstützung der Wartung beliebiger land-, luft-, wasser- oder unterwassergebundener Fahrzeuge verwendet werden kann. Voraussetzung dafür ist, dass eine Sensoreinrichtung zur Erzeugung und/oder Speicherung von Sensorsignalen an zumindest einem Überprüfungsort zur Diagnose von Fehlern an wartungsintensiven Gehäuseteilen des Fahrzeugs vorgesehen ist, wobei die Sensorsignale an eine Verarbeitungseinheit übermittelt werden und die Verarbeitungseinheit mit einer Anzeigeeinrichtung zur Anzeige der Sensorsignale und/oder der ermittelten Diagnoseinformationen in Wirkverbindung steht. Dabei sind die Sensoren und Transceiver in Verschlusselementen zum Verschließen von Ein-/Auslassöffnungen für Schmiermittel in einem Gehäuseteil mit beweglichen Komponenten im Körper des Fahrzeugs integriert ist und der Transceiver im Verschlusselement des Gehäuseteils übermittelt die Sensorsignale aus dem Gehäuseteil an die Verarbeitungseinheit.

## Patentansprüche

1. Verschlussvorrichtung (20) zur Abdichtung einer Öffnung eines Gehäuseteils (10, 30) gegen ein Schmiermittel, die einen Innenraum aufweist, in dem angeordnet ist:
- zumindest eine Sensorvorrichtung (40) mit einem Sensor (41) zur Erzeugung von dem Betriebszustand des Schmiermittels entsprechenden Sensorsignalen,
- zumindest ein Transceiver (50) zum Empfang der Sensorsignale von der Sensorvorrichtung (40) und zur Signalübertragung der Sensorsignale an eine externe Empfangseinheit und
- eine Energieversorgungsvorrichtung (E) zur Versorgung der Sensorvorrichtung (40) und des Transceivers (50) mit elektrischer Energie,
wobei die Oberfläche (31a) eines Endabschnitts (31) als Sensoroberfläche zur Erfassung der Sensorwerte ausgebildet ist,
**dadurch gekennzeichnet, dass** die Sensorvorrichtung (40) einen Planar-Kondensator zur Erfassung des Wassergehalts des Schmiermittels aufweist, der aus zwei einander zugewandten Kondensator-Elektroden (36, 37) gebildet ist.

2. Verschlussvorrichtung (20) nach dem Anspruch 1, **dadurch gekennzeichnet, dass** die Kondensator-Elektroden (36, 37) des Planar-Kondensators (KP) gabelförmig ineinander greifen, um die Größe der einander zugewandten Randlinien oder Randflächen zu maximieren.

3. Verschlussvorrichtung (20) nach einem der voranstehenden Ansprüche, **dadurch gekennzeichnet, dass** der Sensor (41) derart ausgeführt ist, dass dieser einen Messbereich zwischen 0% und 40% relativer Feuchte hat.

4. Verschlussvorrichtung (20) nach einem der voranstehenden Ansprüche, **dadurch gekennzeichnet, dass** die Sensorvorrichtung (40) zusätzlich einen Temperatursensor zur Erfassung der Temperatur des Schmiermittels und/oder einen Drucksensor zur Erfassung des Druckes des Schmiermittels und/oder des Umgebungsdrucks aufweist.

5. Verschlussvorrichtung (20) nach einem der voranstehenden Ansprüche, **dadurch gekennzeichnet, dass** die Verschlussvorrichtung als Schraube und zum Einsetzen in eine Gewindeaufnahme einer Einlassöffnung oder Auslassöffnung oder als Bolzen mit einem Verankerungselement zum Verschließen der jeweiligen Einlassöffnung (41, 43) oder Auslassöffnung (42, 44) mit dem Bolzen ausgebildet ist.

6. Gehäuseteil (10, 30) eines Schmiermittelbehälters, aufweisend einen Innenraum, der insbesondere zur Aufnahme eines Schmiermittels vorgesehen ist, wobei das Gehäuseteil (10, 30) eine Verschlussvorrichtung (20) nach einem der vorhergehenden Ansprüche aufweist.

7. Gehäuseteil (10, 30) nach dem Anspruch 6, **dadurch gekennzeichnet, dass** das einen Schmiermittelbehälter bildende Gehäuseteil (10, 30) ein Gehäuseteil (10, 30) eines Leistungs-Übertragungsmechanismus (11) und insbesondere ein Getriebe ist.

8. Diagnosesystem, aufweisend ein Gehäuseteil (10, 30) nach einem der Ansprüche 6 oder 7 und eine separate Wartungsvorrichtung (W) mit einem Transceiver zum Empfang von Sensorsignalen von dem der Sensorvorrichtung (40) zugeordneten Transceiver (50), wobei die Wartungsvorrichtung eine Verarbeitungsfunktion zur Ermittlung eines Wertes für den Betriebszustand eines in dem Gehäuseteil (10, 30) befindlichen Schmiermittels aus dem Sensorsignal aufweist.

9. Diagnosesystem nach dem Anspruch 8, die Wartungsvorrichtung aufweisend: eine Verarbeitungsfunktion zur Ermittlung eines Wertes für den Betriebszustand eines in dem Gehäuseteil (10, 30) befindlichen Schmiermittels aus dem Sensorsignal,
**dadurch gekennzeichnet, dass** in dem Gehäuseteil (10, 30) mindestens zwei Sensorvorrichtungen (40) und ein diesen zugeordneter Transceiver (50) integriert sind, dass die Wartungsvorrichtung (W) eine Vergleichsfunktion aufweist, die mit dem Transceiver (50) der Wartungsvorrichtung (W) funktional verbunden ist und aus den Sensorsignalen von zwei verschiedenen Sensorvorrichtungen (40) ein Vergleichsfunktionswert gebildet wird, der als Wert für den Betriebszustand eines in dem Gehäuseteil (10, 30) befindlichen Schmiermittels verwendet wird.

10. Diagnosesystem nach dem Anspruch 8 oder 9, **dadurch gekennzeichnet, dass** die Sensorvorrichtung (40) derart eingerichtet ist, dass sie auf Empfang eines Steuerungskommandos von dem der Sensorvorrichtung (40) zugeordneten Transceiver (50) ein einem Betriebszustand des Schmiermittels entsprechendes Sensorsignal erfasst und an die Wartungsvorrichtung (W) sendet.

11. Diagnosesystem nach dem Anspruch 8, 9 oder 10, **dadurch gekennzeichnet, dass** die Sensorvorrichtung (40) aufweist: eine mit der Verarbeitungsfunktion zur Ermittlung eines Wertes für den Betriebszustand eines in dem Gehäuseteil (10, 30) befindlichen Schmiermittels aus dem erfassten Sensorsignal und eine funktional mit der Verarbeitungsfunktion verbundene Diagnosefunktion zur Ermittlung von Wartungsinformationen aus dem jeweils ermittelten Wert für den Betriebszustand eines in dem Gehäuseteil (10, 30) befindlichen Schmiermittels.

12. Diagnosesystem nach einem der Ansprüche 8 bis 11, **dadurch gekennzeichnet, dass** die Wartungsvorrichtung (W) eine Anzeigevorrichtung zur Anzeige der Sensorgrößen und/oder von ermittelten Wartungsinformationen aufweist.

13. Diagnosesystem nach dem Anspruch 12, **dadurch gekennzeichnet, dass** die Wartungsvorrichtung (W) eine mit der Sensorvorrichtung zur Zuführung von Sensorsignalen funktional in Verbindung stehende Vergleichsfunktion aufweist, die derart ausgeführt ist, dass die Vergleichsfunktion das jeweils erfasste Messsignal mit zumindest einem Grenzwert vergleicht und aufgrund dieses Vergleichs identifiziert, ob der jeweils erfasste Signalwert in einem ersten Bereich unterhalb dieses Grenzwerts oder in einem zweiten Bereich oberhalb dieses Grenzwertes gelegen ist, und dass die Wartungsvorrichtung (W) eine Anzeigefunktion aufweist, die aufgrund der Identifizierung eines Bereichs für den jeweils erfassten Signalwert eine Markierung zumindest eines Felds oder eines von zwei Feldern, die jeweils einem der Bereiche zugeordnet sind, in einem Anzeigeformat der Anzeigevorrichtung vornimmt.

14. Diagnosesystem nach dem Anspruch 12, **dadurch gekennzeichnet, dass** die Wartungsvorrichtung (W) eine mit der Sensorvorrichtung zur Zuführung von Sensorsignalen funktional in Verbindung stehende Vergleichsfunktion aufweist, die derart ausgeführt ist, dass die Vergleichsfunktion das jeweils erfasste Messsignal mit zwei Grenzwerten vergleicht und aufgrund dieses Vergleichs identifiziert, ob das jeweils erfasste Signalwert in einem ersten Bereich unterhalb eines ersten Grenzwerts oder in einem zweiten Bereich zwischen dem ersten und einem zweiten Grenzwert, der größer als der erste Grenzwert ist, oder in einem dritten Bereich oberhalb des zweiten Grenzwertes gelegen ist, und dass die Wartungsvorrichtung (W) eine Anzeigefunktion aufweist, die aufgrund der Identifizierung eines Bereichs für den jeweils erfassten Signalwert eine Markierung eines von drei Feldern, die jeweils einem der Bereiche zugeordnet sind, in einem Anzeigeformat der Anzeigevorrichtung vornimmt.

15. Land-, luft-, wasser- oder unterwassergestütztes Fahrzeug, in welches ein Gehäuseteil (10, 30) nach einem der Ansprüche 6 und 7 oder ein Diagnosesystem nach einem der Ansprüche 8 bis 14 eingebaut ist.

## Claims

1. A closure device (20) for sealing an opening of a housing part (10, 30) with respect to a lubricant, which closure device comprises an internal space having arranged therein:
- at least one sensor device (40) comprising a sensor (41) for generating sensor signals corresponding to the operational condition of the lubricant,
- at least one transceiver (50) for receiving the sensor signals from the sensor device (40) and for signal transmission of the sensor signals to an external reception unit, and
- a power supply device (E) for supplying electric power to the sensor device (40) and to the transceiver (50),
wherein the surface (31a) of an end portion (31) is realized as a sensor surface for the detection of the sensor values,
**characterized in that** the sensor device (40) comprises a planar capacitor which is configured to detect the water content of the lubricant and is formed out of two capacitor electrodes (36, 37) facing each other.

2. The closure device (20) according to claim 1, **characterized in that** the capacitor electrodes (36, 37) of the planar capacitor (KP) interdigitate in a fork-shaped manner in order to maximize the amount of the edge lines or edge areas facing each other.

3. The closure device (20) according to one of the preceding claims, **characterized in that** the sensor (41) is realized such that it has a measurement range between 0% and 40% relative humidity.

4. The closure device (20) according to one of the preceding claims, **characterized in that** in addition the sensor device (40) comprises a temperature sensor for detecting the temperature of the lubricant and/or a pressure sensor for detecting the pressure of the lubricant and/or the ambient pressure.

5. The closure device (20) according to one of the preceding claims, **characterized in that** the closure device is realized as a screw and for insertion into a threaded receptacle of an inlet opening or outlet opening or as a bolt with an anchor element configured to close the respective inlet opening (41, 43) or outlet opening (42, 44) by means of the bolt.

6. A housing part (10, 30) of a lubricant container comprising an interior space, which is in particular configured to receive a lubricant, wherein the housing part (10, 30) comprises a closure device (20) according to one of the preceding claims.

7. The housing part (10, 30) according to claim 6, **characterized in that** the housing part (10, 30) forming a lubricant container is a housing part (10, 30) of a power transfer mechanism (11) and is in particular a transmission.

8. A diagnostic system comprising a housing part (10, 30) according to one of claims 6 or 7 and a separate maintenance device (W) with a transceiver for receiving sensor signals from the transceiver (50) associated with the sensor device (40), wherein the maintenance device includes a processing function for determining, from the sensor signal, a value for the operational condition of a lubricant present in the housing part (10, 30).

9. The diagnostic system according to claim 8, the maintenance device comprising: a processing function for determining, from the sensor signal, a value for the operational condition of a lubricant present in the housing part (10, 30),
**characterized in that** at least two sensor devices (40) and a transceiver (50) associated with them are integrated in the housing part (10, 30), that the maintenance device (W) includes a comparison function which is functionally connected to the transceiver (50) of the maintenance device (W), and that a comparison function value is formed on the basis of the sensor signals of two different sensor devices (40), which comparison function value is used as a value for the operational condition of a lubricant present in the housing part (10, 30).

10. The diagnostic system according to claim 8 or 9, **characterized in that** the sensor device (40) is configured such that, in response to reception of a control command from the transceiver (50) associated with the sensor device (40), it detects a sensor signal corresponding to an operational condition of the lubricant and sends it to the maintenance device (W).

11. The diagnostic system according to claim 8, 9 or 10, **characterized in that** the sensor device (40) comprises: a processing function for determining a value for the operational condition of a lubricant present in the housing part (10, 30) on the basis of the detected sensor signal, and a diagnosis function functionally connected to the processing function for determining maintenance information from the respective detected value for the operational condition of a lubricant present in the housing part (10, 30).

12. The diagnostic system according to one of claims 8 to 11, **characterized in that** the maintenance device (W) comprises a display device for displaying sensor values and/or determined maintenance information.

13. The diagnostic system according to claim 12, **characterized in that** the maintenance device (W) comprises a comparison function which is functionally connected to the sensor device for supplying sensor signals, which comparison function is configured in such a manner that the comparison function compares the respective detected measurement signal to at least one limit value and identifies, based on this comparison, whether the respective detected signal value is situated in a first range below this limit value or in a second range above this limit value, and **in that** the maintenance device (W) includes a display function which carries out, based on the identification of a range for the respective detected signal value, a marking of at least one field or of one of two fields, each associated with one of the ranges, in a display format of the display device.

14. The diagnostic system according to claim 12, **characterized in that** the maintenance device (W) comprises a comparison function which is functionally connected to the sensor device for supplying sensor signals, which comparison function is configured in such a manner that the comparison function compares the respective detected measurement signal to two limit values and identifies, based on this comparison, whether the respective detected signal value is situated in a first range below a first limit value or in a second range between the first and a second limit value greater than the first limit value, or in a third range above the second limit value, and **in that** the maintenance device (W) includes a display function which carries out, based on the identification of a range for the respective detected signal value, a marking of one of three fields, each associated with one of the ranges, in a display format of the display device.

15. A land-, air-, water- or underwater-based vehicle, in which is incorporated a housing part (10, 30) according to one of claims 6 and 7 or a diagnostic system according to one of claims 8 to 14.

## Revendications

1. Dispositif de fermeture (20) pour fermer de manière étanche une ouverture d'une partie de boîtier (10, 30) contre un lubrifiant qui présente un espace intérieur dans lequel sont placés :
- au moins un dispositif de capteur (40) avec un capteur (41) pour générer des signaux de capteur qui correspondent à l'état de fonctionnement du lubrifiant,
- au moins un transceiver (50) pour la réception des signaux de capteur du dispositif de capteur (40) et pour la transmission de signal des signaux de capteur à une unité de réception externe et
- un dispositif d'alimentation en énergie (E) pour l'alimentation en énergie électrique du dispositif de capteur (40) et du transceiver (50),
cependant que la surface (31 a) d'une section d'extrémité (31) est configurée comme surface de capteur pour la saisie des valeurs de capteur,
**caractérisé en ce que** le dispositif de capteur (40) présente un condensateur planaire pour la saisie de la teneur en eau du lubrifiant qui est formé par deux électrodes de condensateur tournées l'une vers l'autre.

2. Dispositif de fermeture (20) selon la revendication 1, **caractérisé en ce que** les électrodes de condensateur (36, 37) du condensateur planaire (KP) s'engrènent en forme de fourche l'une dans l'autre pour maximiser la grandeur des lignes de bord ou des surfaces de bord tournées l'une vers l'autre.

3. Dispositif de fermeture (20) selon l'une des revendications précédentes, **caractérisé en ce que** le capteur (41) est réalisé de telle manière qu'il a une plage de mesure entre 0% et 40% d'humidité relative.

4. Dispositif de fermeture (20) selon l'une des revendications précédentes, **caractérisé en ce que** le dispositif de capteur (40) présente en plus un capteur de température pour la saisie de la température du lubrifiant et/ou un capteur de pression pour la saisie de la pression du lubrifiant et/ou de la pression ambiante.

5. Dispositif de fermeture (20) selon l'une des revendications précédentes, **caractérisé en ce que** le dispositif de fermeture est configuré comme une vis et pour l'insertion dans un logement fileté d'une ouverture d'admission ou d'une ouverture d'évacuation ou comme un boulon avec un élément d'ancrage pour fermer l'ouverture d'admission respective (41, 43) ou l'ouverture d'évacuation respective (42, 44) avec le boulon.

6. Partie de boîtier (10, 30) d'un réservoir de lubrifiant qui présente un espace intérieur qui est prévu en particulier pour recevoir un lubrifiant, cependant que la partie de boîtier (10, 30) présente un dispositif de fermeture (20) selon l'une des revendications précédentes.

7. Partie de boîtier (10, 30) selon la revendication 6, **caractérisée en ce que** la partie de boîtier (10, 30) qui forme le réservoir de lubrifiant est une partie de boîtier (10, 30) d'un mécanisme de transmission de puissance (11) et en particulier est un engrenage.

8. Système de diagnostic qui présente une partie de boîtier (10, 30) selon l'une des revendications 6 ou 7 et un dispositif séparé de maintenance (W) avec un transceiver pour la réception de signaux de capteur du transceiver (50) associé au dispositif de capteur (40), cependant que le dispositif de maintenance présente une fonction de traitement pour la détermination d'une valeur pour l'état de fonctionnement d'un lubrifiant qui se trouve dans la partie de boîtier (10, 30) à partir du signal du capteur.

9. Système de diagnostic selon la revendication 8 qui présente le dispositif de maintenance : une fonction de traitement pour déterminer une valeur pour l'état de fonctionnement d'un lubrifiant qui se trouve dans la partie de boîtier (10, 30) à partir du signal de capteur,
**caractérisé en ce qu'**au moins deux dispositifs de capteur (40) et un transceiver (50) qui leur est associé sont intégrés dans la partie de boîtier (10, 30), que le dispositif de maintenance (W) présente une fonction de comparaison qui est liée sur le plan fonctionnel au transceiver (50) du dispositif de maintenance (W) et qu'une valeur de fonction de comparaison, qui est utilisée comme valeur pour l'état de fonctionnement d'un lubrifiant qui se trouve dans la partie de boîtier (10, 30), est formée à partir des signaux de capteur de deux dispositifs de capteur différents (40).

10. Système de diagnostic selon la revendication 8 ou 9, **caractérisé en ce que** le dispositif de capteur (40) est agencé de telle manière qu'il saisit un signal de capteur correspondant à un état de fonctionnement du lubrifiant à la réception d'un ordre de commande du transceiver (50) associé au dispositif de capteur (40) et l'envoie au dispositif de maintenance (W).

11. Système de diagnostic selon la revendication 8, 9 ou 10, **caractérisé en ce que** le dispositif de capteur (40) présente : une fonction de diagnostic avec la fonction de traitement pour déterminer une valeur pour l'état de fonctionnement d'un lubrifiant qui se trouve dans la partie de boîtier (10, 30) à partir du signal de détecteur saisi et qui est liée sur le plan fonctionnel à la fonction de traitement pour déterminer des informations de maintenance à partir de la valeur respectivement déterminée pour l'état de fonctionnement d'un lubrifiant qui se trouve dans la partie de boîtier (10, 30).

12. Système de diagnostic selon l'une des revendications 8 à 11, **caractérisé en ce que** le dispositif de maintenance (W) présente un dispositif d'affichage pour l'affichage de grandeurs de capteur et/ou d'informations de maintenance qui ont été détectées.

13. Système de diagnostic selon la revendication 12, **caractérisé en ce que** le dispositif de maintenance (W) présente une fonction de comparaison qui est relation sur le plan fonctionnel avec le dispositif de capteur pour l'alimentation de signaux de capteur qui est réalisée de telle manière que la fonction de comparaison compare le signal de mesure respectivement saisi à au moins une valeur de seuil et identifie, en raison de cette comparaison, si la valeur de signal respectivement saisie est située dans une première plage au-dessous de cette valeur de seuil ou dans une seconde plage au-dessus de cette valeur de seuil et que le dispositif de maintenance (W) présente une fonction d'affichage qui, en raison de l'identification d'une plage pour la valeur de signal respectivement saisie, effectue un marquage d'au moins un champ ou de deux champs qui sont associés à respectivement l'une des plages dans un format d'affichage du dispositif d'affichage.

14. Système de diagnostic selon la revendication 12, **caractérisé en ce que** le dispositif de maintenance (W) présente une fonction de comparaison qui est relation sur le plan fonctionnel avec le dispositif de capteur pour l'alimentation de signaux de capteurs qui est réalisée de telle manière que la fonction de comparaison compare le signal de mesure respectivement saisi à deux valeurs de seuil et, en raison de cette comparaison, identifie si la valeur de signal respectivement saisie se situe dans une première plage au-dessous d'une première valeur de seuil ou dans une seconde plage entre la première valeur de seuil et une seconde valeur de seuil qui est plus grande que la première valeur de seuil ou est située dans une troisième plage au-dessus de la seconde valeur de seuil et que le dispositif de maintenance (W) présente une fonction d'affichage qui, en raison de l'identification d'une plage pour la valeur de signal respectivement saisie, effectue un marquage d'un de trois champs qui sont respectivement associés à l'une des plages.

15. Véhicule basé sur terre, air, eau ou sous-marin dans lequel une partie de boîtier (10, 30) selon l'une des revendications 6 et 7 est intégrée ou un système de diagnostic selon l'une des revendications 8 à 14.
